# EUROPEAN PATENT APPLICATION

(11) **EP 4 382 067 A2**
(43) Date of publication of application: **12.06.2024**
(21) Application number: 23215360.1
(22) Date of filing: 08.12.2023
(51) Int. Cl.: A61B 34/20, A61B 17/17, A61B 90/00

(54) **SYSTEMS AND METHODS FOR VISUALLY INDICATING DRILLED HOLE POSITION IN ENDOSCOPIC PROCEDURES**

(30) Priority: 09.12.2022 US 202263386890 P
(71) Applicant: Stryker Corporation, Kalamazoo, MI 49002 (US)
(72) Inventor: FOUTS, Brian, Kalamazoo, 49002 (US); HUNTER, Cole Kincaid, Kalamazoo, 49002 (US); GUREVICH, Lina, Kalamazoo, 49002 (US)
(74) Representative: V.O.

(57) **Abstract**

Devices and methods for determining a position of a hole drilled in tissue in endoscopic surgical procedures include a drill guide configured to guide a drill bit to drill a hole in tissue of internal anatomy of a patient, the drill guide comprising: a shaft configured to receive the drill bit, wherein a distal end of the shaft is configured to interface with the tissue; and at least one fiducial marker positioned proximate the distal end of the shaft and configured to provide position information for the hole in an endoscopic image captured by an endoscopic imaging device.

## Description

### CROSS-REFERENCE TO RELATED APPLICATIONS

This application claims the benefit of U.S. Provisional Patent Application No. 63/386,890, filed December 9, 2022, the entire contents of which are hereby incorporated by reference herein.

### FIELD

This disclosure is related generally to endoscopic imaging, and more specifically, to tracking the position of a drilled hole in endoscopic images.

### BACKGROUND

Endoscopic imaging involves the use of a camera coupled to an endoscope inserted into a patient to provide a surgeon with a clear and precise view within the body. Video data collected by the camera is rendered on a display so that the surgeon can visualize the internal area of the body that is being viewed by the camera. The camera can serve as the eyes of the surgeon during the surgery since the camera may provide the only view of the internal area of the patient. In many instances, the surgeon may depend on the camera to perform procedures in the internal area of the patient using one or more tools that are specifically configured for endoscopic procedures to aid the surgeon as they perform the procedure. The surgeon can view the imaging feed being displayed to them during a surgery to manipulate the tool and navigate the tool within the internal area of the patient.

Orthopedics is a medical specialty involving the diagnosis, correction, prevention, and treatment of skeletal conditions, including conditions or disorders involving bones, joints, muscles, ligaments, and tendons. Orthopedic surgical procedures often involve manipulating bony anatomy. Such procedures may include, for example, the placement of anchors in bony anatomy. This often includes drilling a hole into the bony anatomy for insertion of a bone screw or other anchor. To aid in drilling the hole, surgeons may use a drill guide, which is typically positioned against a surface of the bony anatomy into which the hole is to be drilled. The drill guide includes a shaft for receiving and guiding the drill bit. The drill guide can assist in proper positioning and orienting of the drilled hole.

### SUMMARY

According to various aspects, systems and methods are used to visually indicate the placement of a drilled hole in anatomy of interest of a patient in one or more endoscopic images. A distal end of a drill guide positioned against bony anatomy is captured in one or more endoscopic images. The drill guide includes at least one fiducial marker. The fiducial marker is identified in the endoscopic images, and based on the position and orientation of the fiducial marker in the endoscopic images and predetermined positioning of the center of the distal end of the drill guide, the position of a hole drilled by a drill guided by the drill guide is determined. A visual indication may be displayed to the user that includes a graphical indication of the position of the hole in the endoscopic images.

Determining the position of the drilled hole may be performed after first determining that the hole has been drilled. A determination that the hole has been drilled may be made by analyzing the endoscopic images to identify indications that the hole is being drilled or has been drilled. For example, the drill guide may include a window within which a drill bit can be observed and the presence of the drill bit in the window can be detected in the endoscopic imaging. Thus, a graphical indication of a drilled hole can be automatically created, which can guide a surgeon during an endoscopic procedure.

According to an aspect, a drill guide for use in endoscopic surgical procedures that is configured to guide a drill bit to drill a hole in tissue of internal anatomy of a patient includes a shaft configured to receive the drill bit, wherein a distal end of the shaft is configured to interface with the tissue; and at least one fiducial marker positioned proximate the distal end of the shaft and configured to provide position information for the hole in an endoscopic image captured by an endoscopic imaging device.

The shaft may include a window for viewing the drill bit when the drill bit is received in the shaft. The window may be configured for viewing a marker disposed on the drill bit when the drill bit is drilling the hole in the tissue.

The at least one fiducial marker may include at least one ArUco marker. The at least one fiducial marker may include a plurality of fiducial markers. Each fiducial marker of the plurality of fiducial markers may be disposed on a different face of a fiducial ring. Each fiducial marker of the plurality of fiducial markers may be configured to identify which face of the fiducial ring on which the fiducial marker is disposed. The fiducial ring may be coaxial with the shaft. Each fiducial marker of the plurality of fiducial markers may include identifying information that uniquely identifies the respective fiducial marker relative to the other fiducial markers of the plurality of fiducial markers.

According to an aspect, a method for determining a position of a hole drilled in tissue of internal anatomy of a patient by a drill bit disposed within a drill guide includes, at a computing system, receiving at least one endoscopic image that captures at least a distal portion of a shaft of the drill guide; identifying in the at least one endoscopic image at least one fiducial marker disposed on the distal portion of the shaft of the drill guide; determining a position and orientation of the at least one fiducial marker; determining that the drill bit drilled the hole in the tissue; and determining the position of the hole based on the position and orientation of the at least one fiducial marker. It will be appreciated that the endoscope and the drill guide may have been inserted into the subject prior to the start of the method. The drill bit can have drilled the hole in the tissue prior to the start of the method. It will be appreciated that the endoscopic image is received. The step of obtaining the endoscopic image is not part of the method.

The method may further include displaying a visualization of the tissue that includes a marker indicating the position of the hole. Displaying the visualization may include overlaying the marker on an endoscopic video and updating the position of the marker based on changes in the field of view of the endoscopic video. The method may further include modifying the display of the visualization of the tissue based on at least one user input.

Determining that the drill bit drilled the hole in the tissue may include determining from the at least one endoscopic image that the drill bit drilled the hole in the tissue. Determining from the at least one endoscopic image that the drill bit drilled the hole in the tissue may include determining that the drill bit is visible in a window of the shaft. Determining from the at least one endoscopic image that the drill bit drilled the hole in the tissue may include determining that the drill bit has been visible in the window of the shaft for at least a predetermined period. The predetermined period may be a predetermined time period or a predetermined number of endoscopic images. The method may further include locating the window of the shaft in the at least one image based on the position and orientation of the at least one fiducial marker.

Determining from the at least one endoscopic image that the drill bit drilled the hole in the tissue may include determining that a marker on the drill bit is visible in a window of the shaft. Determining from the at least one endoscopic image that the drill bit drilled the hole in the tissue may include detecting debris created by the drill bit drilling the hole in the at least one image.

Identifying the at least one fiducial marker disposed on the shaft of the drill guide may include applying one or more machine learning models to the received endoscopic image to segment the drill guide in the at least one endoscopic image.

Identifying the at least one fiducial marker may include: identifying one or more visual patterns of the at least one fiducial marker; and matching the one or more visual patterns of the at least one fiducial marker to at least one fiducial marker pattern in a predefined list of fiducial marker patterns.

The at least one fiducial marker may be disposed on a face of a fiducial ring. Determining the position of the hole may include: selecting a corner of the face corresponding to the at least one fiducial marker; identifying a position of the selected corner with respect to the drill guide based on the determined position and orientation of the at least one fiducial marker; and determining a three-dimensional distance from the selected corner to a center of a distal end of the drill guide based on the identified position of the selected corner with respect to the drill guide.

Determining the three-dimensional distance from the selected corner to the hole may include: identifying a matching corner corresponding to the selected corner, the matching corner stored in a look-up table comprising a list of fiducial markers and corresponding corners, wherein each entry pertaining to a corner in the look-up table includes a predetermined three-dimensional distance between the corner and a position of the center of the distal end of the drill guide; and extracting the predetermined three-dimensional distance corresponding to the identified matching corner corresponding to the selected corner in the look-up table, wherein the position of the center of the distal end of the drill guide is determined based on the predetermined three-dimensional distance.

In accordance with an aspect, a system for determining a position of a hole drilled in tissue of internal anatomy of a patient by a drill bit disposed within a drill guide, for instance such as described herein, is provided, the system comprising one or more processors, memory, and one or more programs stored in the memory and including instructions for execution by the one or more processors for: receiving at least one endoscopic image that captures at least a distal portion of a shaft of the drill guide; identifying in the at least one endoscopic image at least one fiducial marker disposed on the shaft of the drill guide; determining a position and orientation of the at least one fiducial marker; determining that the drill bit drilled the hole in the tissue; and determining the position of the hole based on the position and orientation of the at least one fiducial marker. The system may comprise the drill guide, such as the drill guide described herein.

The one or more programs may include instructions for displaying a visualization of the tissue that includes a marker indicating the position of the hole. Displaying the visualization may include overlaying the marker on an endoscopic video and updating the position of the marker based on changes in the field of view of the endoscopic video. The one or more programs may include instructions for modifying the display of the visualization of the tissue based on at least one user input.

Determining that the drill bit drilled the hole in the tissue may include determining from the at least one endoscopic image that the drill bit drilled the hole in the tissue. Determining from the at least one endoscopic image that the drill bit drilled the hole in the tissue may include determining that the drill bit is visible in a window of the shaft. Determining from the at least one endoscopic image that the drill bit drilled the hole in the tissue may include determining that the drill bit has been visible in the window of the shaft for at least a predetermined period. The predetermined period may be a predetermined time period or a predetermined number of endoscopic images. The one or more programs may include instructions for locating the window of the shaft in the at least one image based on the position and orientation of the at least one fiducial marker. Determining from the at least one endoscopic image that the drill bit drilled the hole in the tissue includes determining that a marker on the drill bit is visible in a window of the shaft. Determining from the at least one endoscopic image that the drill bit drilled the hole in the tissue may include detecting debris created by the drill bit drilling the hole in the at least one image.

Identifying the at least one fiducial marker disposed on the shaft of the drill guide may include applying one or more machine learning models to the received endoscopic image to segment the drill guide in the at least one endoscopic image.

Identifying the at least one fiducial marker may include identifying one or more visual patterns of the at least one fiducial marker; and matching the one or more visual patterns of the at least one fiducial marker to at least one fiducial marker pattern in a predefined list of fiducial marker patterns.

The at least one fiducial marker may be disposed on a face of a fiducial ring. Determining the position of the hole may include selecting a corner of the face corresponding to the at least one fiducial marker; identifying a position of the selected corner with respect to the drill guide based on the determined position and orientation of the at least one fiducial marker; and determining a three-dimensional distance from the selected corner to a center of a distal end of the drill guide based on the identified position of the selected corner with respect to the drill guide. Determining the three-dimensional distance from the selected corner to the hole may include identifying a matching corner corresponding to the selected corner, the matching corner stored in a look-up table comprising a list of fiducial markers and corresponding corners, wherein each entry pertaining to a corner in the look-up table includes a predetermined three-dimensional distance between the corner and a position of the center of the distal end of the drill guide; and extracting the predetermined three-dimensional distance corresponding to the identified matching corner corresponding to the selected corner in the look-up table, wherein the position of the center of the distal end of the drill guide is determined based on the predetermined three-dimensional distance.

It will be appreciated that any of the variations, aspects, features and options described in view of the systems apply equally to the methods and vice versa. It will also be clear that any one or more of the above variations, aspects, features and options can be combined.

### BRIEF DESCRIPTION OF THE FIGURES

The invention will now be described, by way of example only, with reference to the accompanying drawings, in which:
FIG. 1 illustrates an exemplary endoscopy system;
FIG. 2 is an example of a visualization that may be generated for assisting a surgeon or other user in tracking the position of a drilled hole;
FIG. 3 illustrates an example of a drill guide that includes a fiducial marker for determining a position of a hole drilled in one or more endoscopic images;
FIGS. 4A and 4B illustrates examples of arrangements of a plurality of fiducial markers on drill guides;
FIGS. 4C and 4D illustrate an exemplary ArUco marker that can be used as the fiducial marker of a drill guide;
FIG. 5 is a flowchart of an exemplary method for determining the position of a hole drilled in tissue of internal anatomy of a subject by a drill bit disposed within a drill guide;
FIG. 6 illustrates examples of various visual indications associated with hole drilling that may be detected in one or more endoscopic images for automatically determining that a hole has been drilled;
FIG. 7 illustrates an exemplary visualization of tissue of interest in which one or more graphical indications of one or more holes drilled in the tissue are relocated in the visualization based on movement of the endoscopic imager such that the graphical indication(s) continues to accurately indicate the position of the hole(s) relative to the displayed scene;
FIG. 8 illustrates an exemplary method for repositioning a graphical indication of a hole drilled in tissue in a visualization according to movement of an endoscopic imager;
FIG. 9 illustrates an example of a computing system;
FIG. 10 illustrates an exemplary use of a drill guide;
FIG. 11 is a flow diagram of an exemplary method for estimating motion of an endoscopic imager between frames;
FIG. 12 illustrates and exemplary key frame cloud used for tracking motion of an endoscopic imager; and
FIG. 13 is a flow diagram of an exemplary method for building a key frame cloud.

### DETAILED DESCRIPTION

Reference will now be made in detail to implementations and embodiments of various aspects and variations of systems and methods described herein. Although several exemplary variations of the systems and methods are described herein, other variations of the systems and methods may include aspects of the systems and methods described herein combined in any suitable manner having combinations of all or some of the aspects described.

Described herein are systems and methods for determining the position in endoscopic images (single snapshot images and/or video frames) of one or more holes drilled into internal anatomy of a subject. A drill guide used to guide a drill bit for drilling the hole(s) is captured in an endoscopic image. The drill guide includes at least one fiducial marker. The fiducial marker is identified in the endoscopic image and its position and orientation are determined. The position and orientation of the fiducial marker(s) can be used in combination with a predetermined position of a distal end of the drill guide relative to the fiducial marker or relative to a feature of the drill guide associated with the fiducial marker to determine where a hole drilled using the drill guide is located in the endoscopic image. The determined position of the hole in the endoscopic image is used to create a visualization that indicates the position of the hole. For example, the visualization can include the endoscopic image with a flag or other graphical indication overlayed on the endoscopic image at the position of the hole.

Systems and methods may include automatic detection that a hole has been drilled so that a graphical indication of the hole is displayed once the hole has been drilled. The automatic detection that a hole has been drilled may include detecting indications that a hole is being drilled or has been drilled based on one or more endoscopic images. Examples of such indications include the positioning of the drill guide against tissue, the positioning of a drill bit in the drill guide, the appearance of debris resulting from drilling in the endoscopic images, and the ceasing of one or more of these. The automatic detection that a hole has been drilled can be done using a machine learning model trained to detect a hole drilling operation.

The systems and methods described herein can help guide a user, such as a surgeon, during a surgical procedure on a patient. Once a hole has been drilled, it may be difficult in some instances for the surgeon to keep track of the position of the hole. For example, the surgeon may drill more than one hole prior to placing anchors in the holes and it may be difficult to track each hole, or the surgeon may move the endoscopic imager for one reason or another and the limited field of view of endoscopic imagers leaving the field of view for some period of time and the surgeon may have difficulty relocating the hole. The systems and methods described herein can make tracking the hole placement much easier by providing a graphical indication of the hole(s) overlayed on an endoscopic image (e.g., endoscopic video feed). The graphical indication may be repositioned as the field of view the endoscopic imager changes.

In the following description of the various embodiments, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes, "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating" or the like, refer to the action and processes of a computer system, or similar electronic computing device, that manipulates and transforms data represented as physical (electronic) quantities within the computer system memories or registers or other such information storage, transmission, or display devices.

The present disclosure in some embodiments also relates to a device for performing the operations herein. This device may be specially constructed for the required purposes, or it may comprise a general purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each connected to a computer system bus. Furthermore, the computing systems referred to in the specification may include a single processor or may be architectures employing multiple processor designs, such as for performing different functions or for increased computing capability. Suitable processors include central processing units (CPUs), graphical processing units (GPUs), field programmable gate arrays (FPGAs), and ASICs.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present disclosure as described herein.

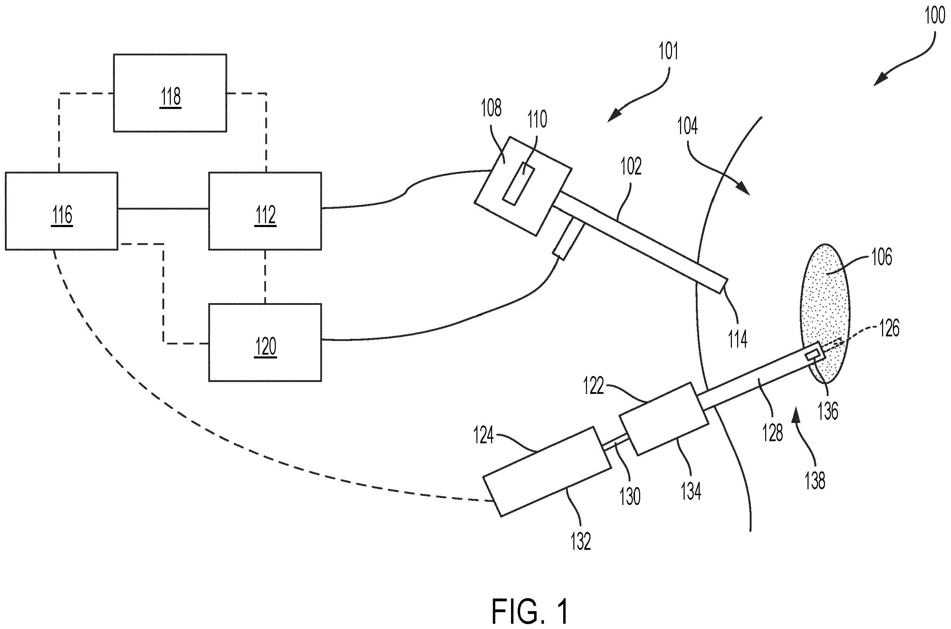

FIG. 1 illustrates an exemplary endoscopy system. System 100 includes an endoscopic imager 101 that can include a camera head 108 mounted to an endoscope 102. The endoscope 102 can be configured for insertion into a surgical cavity 104 for imaging tissue 106 within the surgical cavity 104 during a medical procedure. The endoscopic camera head 108 includes one or more imaging sensors 110. Light generated by a light source 120 may be directed through the endoscope 102 to the surgical cavity 104. Light reflected by and/or emitted from the tissue 106 (such as fluorescence light emitted from fluorescing targets that are excited by fluorescence excitation illumination light provided the light source 120) is received at the distal end 114 of the endoscope 102. The light is propagated by the endoscope 102, such as via one or more optical components (for example, one or more lenses, prisms, light pipes, or other optical components), to the camera head 108, where it is directed onto the one or more imaging sensors 110. One or more filters (not shown) may be included in the endoscope 102, in a coupler (not shown) connecting the endoscope 102 to the camera head 108, and/or in the camera head 108 for filtering a portion of the light received from the tissue 106 (such as fluorescence excitation light).

The one or more imaging sensors 110 generate pixel data that can be transmitted to a camera control unit 112 that is communicatively connected to the camera head 108. The camera control unit 112 can generate endoscopic images (as used herein, "endoscopic image(s)" encompasses single snapshot images and/or a sequence of video frames) from the pixel data that shows the tissue being viewed by the endoscopic imager 101. The endoscopic images can be transmitted to an image processing system 116 for further image processing, storage, display, and/or routing to an external device (not shown). The image processing system 116 receives the endoscopic images from the camera control unit 112. The endoscopic images can be transmitted to one or more displays 118, from the camera control unit 112 and/or the image processing system 116, for visualization by medical personnel, such as by a surgeon for visualizing the surgical cavity 104 during a surgical procedure on a patient. The camera control unit 112 and/or the image processing system 116 may be configured to send control signals to the light source 120 and/or the camera head 108 to control one or more aspects of the imaging, such as a timing sequence of light provided by the light source 120 (e.g., a sequence of white light and fluorescence excitation light), an amount of light provided by the light source 120, and/or a gain of the one or more imaging sensors 110.

One or more surgical tools may be used in the surgical cavity 104 to manipulate tissue 106 during a surgical procedure on the patient, and the surgical tools may be captured in the images captured by the camera head 108 and displayed on the display 118 so that the medical personnel can view the interaction between the one or more surgical tools and the tissue. For example, the tissue 106 may be bone, and the one or more tools may include a drill guide 122 used for guiding a drill 124 to drill a hole 126 in the bone. A surgeon may position the drill guide 122 against the tissue 106 at a desired position and may orient the drill guide 122 according to a desired orientation for the hole 126. The drill guide 122 includes a shaft 128 for receiving and guiding a drill bit 130 of the drill 124. The surgeon may activate the drill 124 and advance the drill bit 130 into the tissue 106 to drill the hole 126. The surgeon may advance the drill bit 130 until a proximal portion 132 of the drill 124 abuts a proximal portion 134 of the drill guide 122, which stops advancement of the drill bit 130 within the tissue 106, thereby controlling the depth of the hole 126. The drill 124 may be communicatively connected to the image processing system 116, which may enable a user to indicate a desire for the image processing system 116 to determine a position of the hole 126 in an endoscopic image.

After the hole 126 has been drilled, the surgeon may move the drill guide 122 away from the hole 126, such as for observing the hole 126, for driving a bone screw or other type of anchor or device into the hole 126, for repositioning the drill guide 122 for drilling other holes, or for any other reason. During post-hole drilling activity, it may be difficult for the surgeon to keep track of the hole 126 (e.g., due to the limited field of view provided by the endoscope 102, obstructed field of view due to surrounding tissue, movement of the endoscope 102, and the like). To assist the surgeon in locating the hole 126, system 100 can be configured to display a graphical indication to the user indicating where the hole is located relative to the displayed endoscopic images. As described further below, the system 100 may analyze one or more endoscopic images to determine where the hole 126 is located relative to the endoscopic imager 101 and may generate and display a visualization (e.g., on display 118) that includes a graphical indication of the position of the hole 126 relative to a displayed endoscopic image.

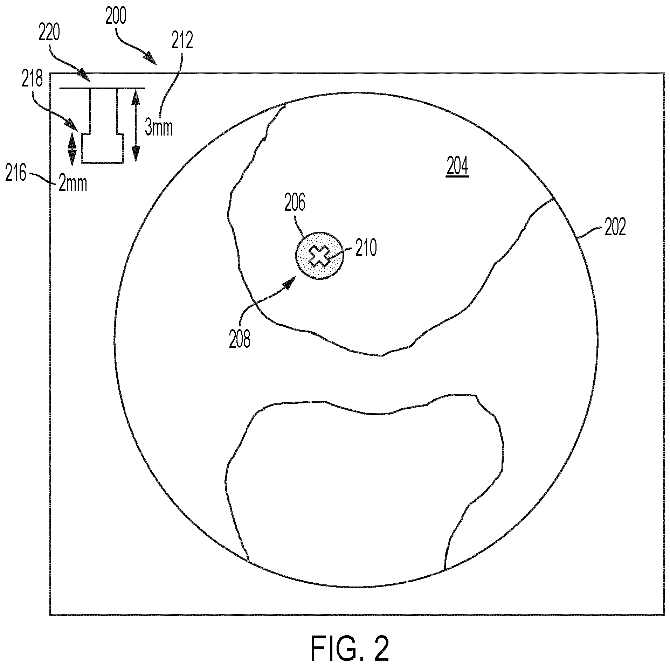

FIG. 2 is an example of a visualization that may be generated by the image processing system 116 and displayed on the display 118 for assisting a surgeon or other user in tracking the position of a drilled hole. The visualization 200 includes an endoscopic image 202 of internal anatomy of a subject. The endoscopic image 202 may capture bony anatomy 204, or any other type of anatomy, into which a hole 206 has been drilled. The visualization 200 includes a graphical indication 208 of the position of the hole 206 relative to the displayed endoscopic image 202. The graphical indication 208 can include, for example, an indication 210 of a center of an entrance to the hole 206, indicated by a marker (e.g., a flag, "X", etc.).

Returning to FIG. 1, a visualization such as visualization 200 may be generated by the image processing system 116, which may analyze one or more endoscopic images received from and generated by the endoscopic imager 101 to determine the position of the hole 126. As discussed in more detail below, the image processing system 116 may use the drill guide 122 as a guide for where the hole 126 is located in the one or more endoscopic images. To this end, the drill guide 122 may include at least one fiducial marker 136 that may be captured in the endoscopic images. The fiducial marker 136 may be identified by the image processing system 116 and predefined information associated with the fiducial marker 136 may be utilized by the image processing system 116 to determine where the hole 126 is located in the endoscopic images. For example (and as discussed more fully below), characteristics of the fiducial marker 136 in the endoscopic image(s) may be compared to predetermined characteristics of the fiducial marker 136 to determine the position and orientation of the fiducial marker relative to a given reference coordinate system, such as a camera-based coordinate system. The camera-based coordinate system may be defined as a coordinate system with a known origin located relative to the camera (e.g., camera head 108) of the endoscopic imaging system. Thus, the position and orientation of the fiducial marker may be determined relative to the origin located at the camera head. Examples of other reference coordinate systems that may be used include a tool-based coordinate system and a world-based coordinate system. A reference tool-based coordinate system may have as its origin a particular location on, within, or otherwise relative to drill guide 122, such as a center of a distal end of the drill guide 122. A reference world-based coordinate system may have an arbitrarily defined origin that is fixed in world space.

One or more predetermined relationships between the fiducial marker 136, or feature of the drill guide 122 associated with the fiducial marker 136, and a center of a distal end of the drill guide 122 may be used determine a position of the center of the distal end of the drill guide 122 relative to, e.g., the camera-based coordinate system. The position of the center of the distal end of the drill guide 122 may then be used as the position of the hole 126 given the close proximity between the center of the distal end of the drill guide 122 and the hole 126. With the position of the hole 126 determined relative to the reference coordinate system, the image processing system 116 can track where the position of the hole 126 is located relative to endoscopic images generated by the endoscopic imager 101 and can generate a visualization, such as visualization 200 of FIG. 2, that includes a graphical indication of the position of the hole 126 displayed within the endoscopic image.

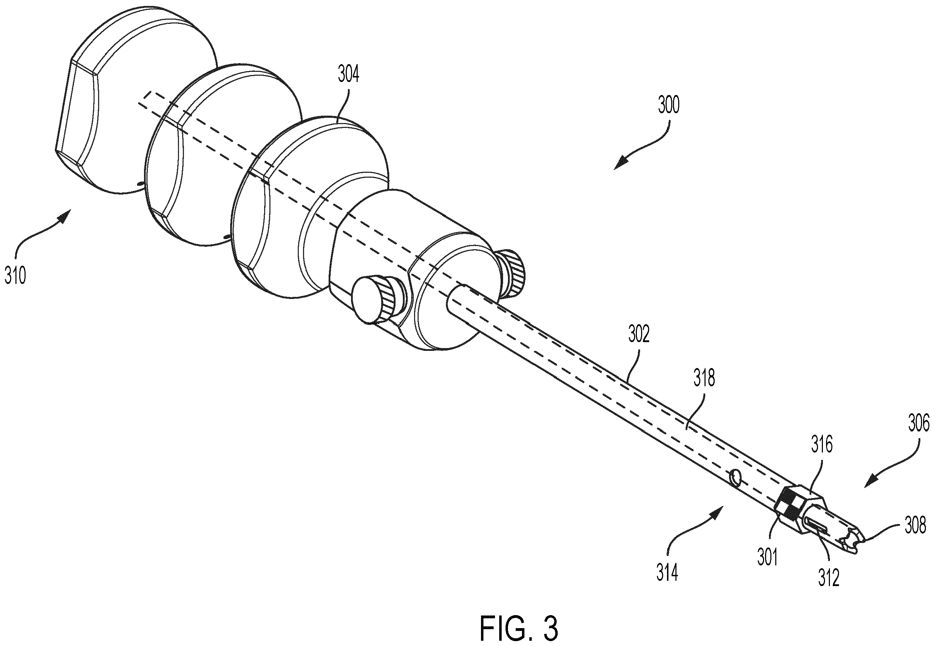

FIG. 3 illustrates an example of a drill guide 300 that includes a fiducial marker 301 that can enable an image processing system, such as image processing system 116, to determine a position of a hole drilled using a drill guided by the drill guide 300 in one or more endoscopic images. The drill guide 300 includes a shaft 302 for insertion into a surgical cavity and a handle 304 for grasping by a user. A distal end 306 of the shaft 302 is configured for placement against tissue, such as bone. The distal end 306 may include one or more teeth 308 or other type of protruding structures that help the distal end 306 remain in position against the tissue. A lumen 318 extends from the proximal end 310 of the handle 304 to the distal end 306 of the shaft 302. A drill bit of a drill is inserted into the lumen 318 and advanced out the distal end 306 of the shaft 302 for drilling into tissue. The shaft 302 may include one or more windows 312 for observing a drill bit located in the shaft 302.

The fiducial marker 301 can be located in the distal portion 314 of the shaft 302 such that the fiducial marker 301 can be captured in endoscopic imaging of the surgical cavity. The fiducial marker 301 can be formed on the shaft 302 itself or can be located on a separate component that is mounted on the shaft 302. For example, the fiducial marker 301 can be located on a ring 316 that is mounted to the distal portion 314 of the shaft 302. Multiple fiducial markers 301 may be provided, such as to increase the likelihood that a fiducial marker will be in the field of view of the endoscopic imager 101 and/or to improve the accuracy of the hole detection process by enabling multiple estimates of the position of the distal end 306 of the shaft 302 in endoscopic images.

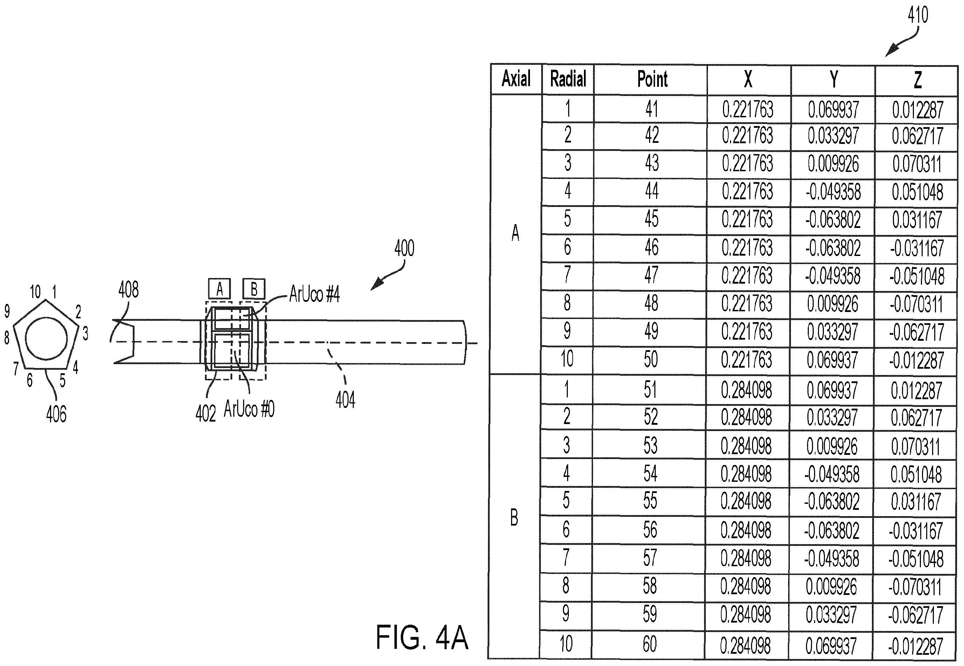

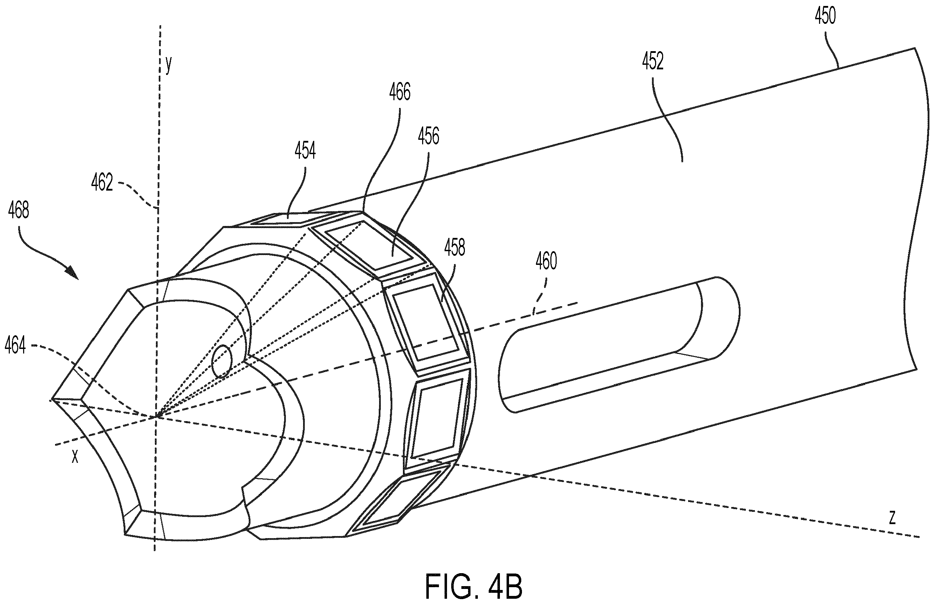

FIGS. 4A and 4B illustrate examples of arrangements of a plurality of fiducial markers of drill guides and how those fiducial markers may be used by an image processing system to determine a position of a center of a distal end of the drill guide and, thereby, the position of a hole drilled by a drill guided by the drill guide. As described further below, each fiducial marker can have predetermined characteristics that may be used by an imaging processing system to identify the fiducial marker. For example, the image processing system may use an edge detection technique to locate intersecting straight lines in an endoscopic image to identify the fiducial marker, or the image processing system may use some other pattern matching technique, such as a machine learning model trained to identify the fiducial marker. The relative positions of one or more features of the fiducial marker (e.g., the position and orientation of detected patterns) may be compared to predetermined spatial relationships of (e.g., distances between) those features to determine the positions of those features relative to a reference coordinate system, such as a camera-based coordinate system, and thereby, the position and orientation of the fiducial marker, of the drill guide, and/or of any feature of the drill guide.

FIG. 4A illustrates an exemplary drill guide 400 that includes a fiducial ring 402. The illustrated fiducial ring 402 includes 5 sides, thus making the fiducial ring 402 a pentagon that is "wrapped around" the axis 404 of the drill guide 400 that the fiducial ring 402 is disposed on. Cross-section 406 illustrates a cross-sectional view of fiducial ring 402. Cross-section 406 illustrates that the pentagonal shape of the fiducial ring 402 includes 5 faces. The 5 faces collectively form two separate sets of 10 corners. As shown in FIG. 4A, the two separate sets of 10 corners are labeled "A" and "B" and the corners are labeled numerically in cross-section 406.

Each corner of the fiducial ring 402 may be disposed a predetermined distance from the center 408 of the distal end of the drill guide 400 such that when the position of a corner is determined in an endoscopic image (e.g., by image processing system 116), the position of the center 408 can be automatically derived based on the predetermined distance. The distance from each corner to the center 408 can be empirically measured at the time of manufacture of the drill guide 400. When the drill guide 400 is in use during a surgical procedure, an image processing system analyzing endoscopic images that include the drill guide can have beforehand knowledge about the predetermined distance relationships between each corner of the fiducial ring 402 and the center 408. In one or more examples, the distance from a corner to the center 408 can be based on a Cartesian coordinate system, such that the center 408 of the drill guide 400 is designated as the origin (i.e., (0,0,0)). Thus, in one or more examples, the distance of each corner (e.g., corners 1-10) of each separate set of corners ("A" and "B") can be measured in terms of displacement in a particular coordinate axis from the center 408. Table 410 illustrates an exemplary table of distances for the corners of the two separate sets of corners ("A" and "B").

In an example entry of table 410, The set of corners denoted as "A" and specifically corner "1" of ring A can have a three-dimensional coordinate (e.g., (x,y,z)) entry that represents the distance in that particular coordinate plane from the corner to center 408. Thus, in the example of A-1, the corner associated with that entry is 0.221763 inches away from the center 408 in the X-direction, 0.069937 inches away from the center 408 in the Y-direction, and 0.012287 inches away from the center 408 in the Z-direction. Thus, in one or more examples, if a three-dimensional position of a corner is determined (described in detail below), then the entries in table 410 corresponding to the corner can be added and/or subtracted to the three-dimensional position of the corner to derive the position of the center 408 of drill guide 400.

In one or more examples, a fiducial marker can be placed on each face of the fiducial ring to aid an image processing algorithm to determine the three-dimensional position of one or more corners associated with the fiducial marker. In the example of FIG. 4A, a fiducial ring with corners is disclosed as an example feature that can be used to determine the position of the center 408, however the example should not be seen as limiting. In one or more examples, the fiducial markers can be used to identify the position of any feature associated with the drill guide that has a known three-dimensional distance from the center 408 such that when the position of the feature is identified, a corresponding three-dimensional distance can be added and/or subtracted to the determined position in order to determine the position of the center 408.

FIG. 4B illustrates another example of a drill guide having a fiducial ring with a plurality of fiducial markers. Drill guide 450 includes a shaft 452 and fiducial ring 454 that is coaxial with the shaft 452. The fiducial ring 454 can include a plurality of faces 456 that are uniformly positioned about a longitudinal axis 460 of the shaft 452. Each face 456 may include a fiducial marker 458. In the illustrated example, the fiducial marker 458 is a square that contrasts with the background (e.g., white on black, black on white, etc.), but this is merely an example of a type of fiducial marker that can be used. This arrangement of fiducial markers 458 can help ensure that at least one fiducial marker 458 is within the field of view of an endoscopic imager when in a surgical cavity. The illustrated fiducial ring 454 includes ten faces. However, this is not intended to be limiting as a fiducial ring could have a fewer or greater number of faces. The fiducial ring 454 may be a separate component mounted to the shaft 452, as shown, or the fiducial ring could be formed (e.g., machined) from the same piece of material as the shaft 452. The fiducial ring may comprise a polygonal shape (such as shown in FIGS 4A & 4B), circular shape, and/or any other shape suitable for displaying fiducial markers within the field of view of the camera. In some embodiments, the shaft 452 may comprise more than one fiducial ring, each fiducial ring comprising one or more fiducial markers.

FIG. 4B includes an exemplary coordinate system 462 for drill guide 450. The center 464 of the distal end of the shaft 452 can correspond to the origin (0,0,0) of the coordinate system 462. A database, such as table 410 of FIG. 4A, listing predefined distances from the corners 466 of each face 456 to the center 464 can include the distances along the x-axis, y-axis, and z-axis from the center 464, as represented by the lines connecting the corners 466 to the center 464 in FIG. 4B. This illustrates that if a three-dimensional position of any one or more of the corners is determined, the position of the center 464 can be determined using a database such as table 410.

In one or more examples, each face of a fiducial ring can have a fiducial marker disposed on it that identifies the specific face of the plurality of faces of the fiducial ring that the fiducial marker is located on as well as the position of the corners of a given face on the fiducial ring. Using these identified attributes of a fiducial marker, the position of the center of the distal end of the drill guide can be ultimately determined as described above. In one or more examples, the fiducial marker disposed on each fiducial ring face can be an ArUco marker. As described below, an ArUco marker can provide a visible pattern that can be efficiently found in an endoscopic image and can provide the information needed to determine the three-dimensional position in space of the center of the distal end of the drill guide.

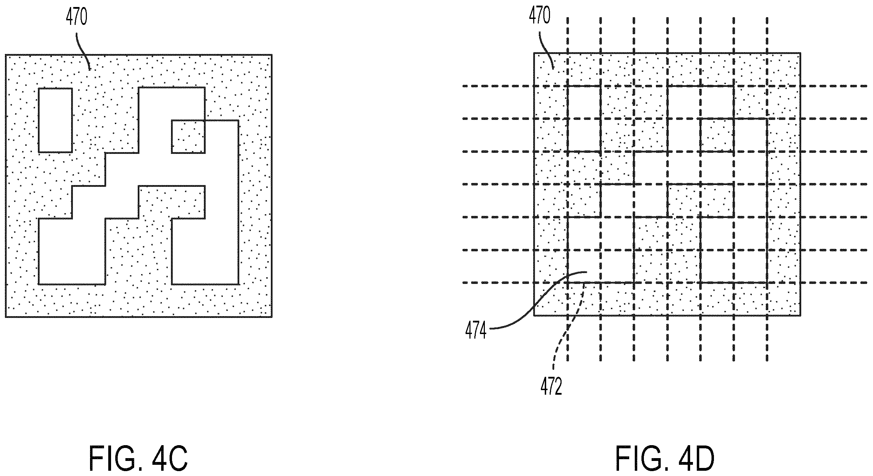

FIGS. 4C and 4D illustrate an exemplary ArUco marker. ArUco marker 470 can include a plurality of black and white blocks arranged in a specific pattern that allows the ArUco marker 470 to not only be identified, but to also be distinguished from other ArUco markers. The blocks of the ArUco marker 470 are arranged in a grid. FIG. 4D illustrates ArUco marker 470 with a grid superimposed on the marker to better illustrate the plurality of blocks 474. In one or more examples, the ArUco marker 470 can contain 64 blocks that are arranged on an 8x8 matrix. In one or more examples, when viewing an ArUco marker 470, an image processing system can extract its four corner points, which enables calculation of its position in three-dimensional space. Each ArUco marker 470 on the drill guide can be unique and can correspond to one position on the drill guide for differentiation.

In one or more examples, the ArUco marker 470 can include a border 472 that frames the marker with a black border. In one or more examples, the border 472 can be disposed on the first row, the last row, the first column, and the last column of the ArUco marker 470. In the example of an 8x8 matrix, the border 472 can be arranged as described above so as to leave an internal 6x6 matrix. In one or more examples, each block 474 of the internal 6x6 matrix can have either a black or white block placed on it. The examples of an 8x8 matrix and 6x6 internal matrix are meant as examples only and should not be seen as limiting to the disclosure. Thus, in one or more examples, a particular ArUco marker 470 can be configured in a variety of dimensions and grid layouts without departing from the scope of the present disclosure.

In one or more examples, the white and black blocks can be arranged on the 6x6 internal matrix so as to provide each ArUco marker 470 with a unique pattern (e.g., bit pattern) which can be used to identify each face of the fiducial ring on the drill guide shaft. In one or more examples, an image processing system can identify the pattern of the ArUco marker 470 and obtain the position of the corners of the ArUco marker 470 (i.e., the corners of the 8x8 matrix). The image processing system can use the determined position of the corners to then find the position of the center of the distal end of the drill guide shaft as described above with respect to FIGS. 4A and 4B. In one or more examples, the ArUco marker 470 of a particular face can be used to determine the orientation of the drill guide. Thus, not only can the ArUco marker 470 be identified, but the orientation of the ArUco marker 470 can be determined to thereby determine the orientation of the drill guide, which can be used to determine the position of the drill guide or a portion of the drill guide. In one or more examples, the ArUco markers can be laser etched (e.g., using an ultraviolet (UV) spectrum laser apparatus) on to each face of the fiducial rings. Additionally or alternatively, the ArUco markers can be printed, machined, or affixed to the face of the fiducial ring using other processes and methods. In some embodiments, the bit patterns may be encoded with error correction that can allow for more robust detection with fewer false positives. For example, in detecting the bit pattern on the fiducial marker(s), errors and/or discrepancies that may occur in detecting each bit may be alleviated using error correction techniques that are standard to and encoded with the fiducial markers to correctly identify the corresponding information associated with a given fiducial marker. Other examples of fiducial markers that may be used are bar codes, Quick Response (QR) codes, AprilTags, and/or any other type of coded marker suitable for providing location information.

Optionally, the positions of one or more features of a fiducial marker (rather than the corners of a face upon which the fiducial marker is located) can be used directly to determine the position of the center of the distal end of the drill guide. For example, features such as one or more points along a perimeter of the fiducial marker (e.g., corners, midpoints, etc.), a center point of the fiducial marker, etc. may be used to determine the center of the distal end of the drill guide. For example, with reference to FIG. 4B, the positions of the corners 466 of fiducial marker 458 can be used to determine the positions of the center 464. A database, such as table 410 of FIG. 4A, may include distances of each corner of a fiducial marker to the center. The corners can be located in an endoscopic image and their positions in the image determined in a reference coordinate system (for example, the three-dimensional positions in a camera-based coordinate system). Once the positions of the corners of the fiducial marker have been determined, the position of the center of the distal end of the drill guide may be determined based on predefined distances between those corners and the center of the distal end of the drill guide, which may be stored in a database (similar to table 410). For example, the position of the center 464 of the distal end 468 of the shaft 452 of the drill guide 450 relative to each corner 466 of the fiducial marker(s) 458 (as indicated by the lines connecting the center 464 to the corners 466 in FIG. 4B) may be predefined and these relative positions may be combined with the positions of the corners 466 in the reference coordinate system (e.g., camera-based coordinate system) to determine the position of the center 412 relative to the reference coordinate system. A transformation matrix (matrix multiplication) may be used to map the center of the drill guide in a tool-based coordinate system (e.g., the position of the center of the distal end of the drill guide relative to one or more features of a fiducial marker or a face upon which a fiducial marker is disposed) to a camera-based coordinate system used to determine the position of the drill guide in relation to the tissue of the patient.

A database, such as table 410 of FIG. 4A, may include information associated with other aspects of the drill and/or drill guide that may be used by the image processing system to determine the position of a hole drilled in tissue. For example, the database may indicate whether the drill guide is curved and/or a degree of curvature, may include an indication of the type of distal end of the drill guide (e.g., fish mouth, crown, etc.), and/or may include an offset associated with a drill bit within the drill guide. Including such indications in the database may be advantageous in determining the position of the hole in tissue with increased accuracy, such as by accounting for offset and/or curvature of drill guide.

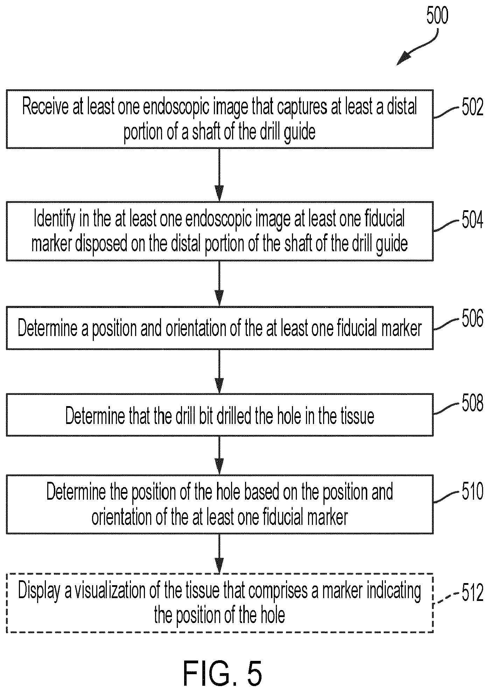

FIG. 5 is a flowchart of a method 500 for determining the position of a hole drilled in tissue of internal anatomy of a subject by a drill bit disposed within a drill guide, such as drill guide 122 or drill guide 300, based on the position and orientation of at least one fiducial marker of the drill guide. Method 500 can be performed by an image processing system, such as image processing system 116 of system 10 of FIG. 1. Method 500 can be performed during a surgical procedure on a subject to provide guidance to one or more surgeons or other medical personnel during the surgical procedure.

At step 502, at least one endoscopic image is received at the image processing system. For example, with respect to FIG. 1, one or more endoscopic images (e.g., a single snapshot image and/or a sequence of video frames) generated by camera control unit 112 based on pixel data from the endoscopic imager 101 may be received by image processing system 116. The endoscopic imager can be pre-inserted prior to start of method 500. The endoscopic image(s) capture tissue of interest within a surgical cavity, such as tissue 106 within surgical cavity 104 of FIG. 1. The tissue of interest may be, for example, bony anatomy. The tissue of interest may be tissue into which a hole has been drilled, is being drilled, or will be drilled. The endoscopic image(s) may also capture a distal portion of a drill guide that is located in the surgical cavity. The drill guide can be pre-inserted prior to start of method 500. For example, with respect to FIG. 1, a distal portion 138 of drill guide 122 may be located in the surgical cavity 104 within a field of view of the endoscopic imager 101. The distal end of the drill guide may be positioned against the tissue of interest, such as for guiding a drill bit to drill a hole in a desired position of the tissue of interest. For example, with respect to FIG. 3, the teeth 308 of the distal end 306 of the drill guide 300 may be positioned against bony anatomy.

One or more fiducial markers located at the distal portion of the drill guide may be captured in the endoscopic image(s). For example, with respect to FIG. 1, fiducial marker 136 may be within the field of view of endoscopic imager 101 such that the fiducial marker 136 is captured in endoscopic image(s) generated by the endoscopic imager 101. More than one fiducial marker may be captured in the endoscopic image(s), such as more than one of the multiple fiducial markers 458 of drill guide 450 of FIG. 4B. The at least one endoscopic image may be a series of frames of a video feed. The steps described below may be performed on each frame of the video feed or may be performed on a subset of the frames of the video feed.

At step 504, at least one fiducial marker of the drill guide is identified in the at least one endoscopic image. The fiducial marker can be identified by detecting one or more visual patterns of the at least one fiducial marker and matching the one or more visual patterns to at least one fiducial marker pattern in a predefined list of fiducial marker patterns. The one or more visual patterns can be detected according to known image processing techniques, such as using a suitable edge detection algorithm and searching for edges that correspond to features of pattern. The fiducial marker can be identified using a machine learning model trained to identify one or more fiducial markers in endoscopic imaging. Identification of the fiducial marker in step 504 may include extraction of information encoded by the fiducial marker. For example, the fiducial marker may be an ArUco marker that encodes an identity of the ArUco marker that may uniquely identify the ArUco marker relative to other ArUco markers such that the image processing system can access the predetermined spatial information associated with that ArUco marker (or the feature(s) of the drill guide associated with that ArUco marker). As described above, the ArUco marker may additionally comprise encoded error detection and correction information such that any discrepancies and/or errors in detecting the bit pattern of the ArUco marker may be minimized to determine the correct information associated with a given ArUco marker.

One or more machine learning models can be used to improve the accuracy of finding and identifying the fiducial markers with respect step 504. For example, a machine learning model can be used to segment the drill guide in the endoscopic image. By segmenting the endoscopic image (i.e., determining the region of the endoscopic image that includes the drill guide), an image processing algorithm can focus on the segmented region to search for the fiducial marker so that the fiducial marker can be more easily found. In one or more examples, a sequence of endoscopic images (a sequence of endoscopic video frames) can be analyzed by one or more machine learning models so that, for instance, any temporal aspects of the video can be used by the one or more machine learning models to segment the tool from an image and/or otherwise help to locate and identify the fiducial markers. In one or more examples, the one or more machine learning models can be trained using images and/or videos of the drill guide that are annotated with the precise position of the drill guide in the image. Thus, the machine learning model(s) may have the ability to recognize the presence and position of a drill guide in a given endoscopic image.

At step 506, a position and orientation of at least a portion of the at least one fiducial marker in the at least one endoscopic image is determined. As discussed above with respect to the fiducial markers 458 of FIG. 4B, the positions of different portions of the fiducial marker relative to one another in the image (e.g., the pixel positions) can be used in combination with predetermined spatial information regarding the positions of those portions relative to one another to determine the position and orientation of the fiducial marker (or portions of the fiducial marker) relative to a reference coordinate system, such as a camera-based coordinate system. For example, the relative positions of the corners 466 of fiducial marker 458 of FIG. 4B in an endoscopic image can be used in combination with the predetermined distance between those corners to determine the three-dimensional positions of the corners 466 (or any other portion(s) of the fiducial marker 458) relative to a camera-based coordinate system.

With reference to system 100 of FIG. 1, the predetermined spatial information regarding the fiducial marker may be stored in a memory of the image processing system 116 or may be stored in a memory of a computing device to which the image processing system 116 is communicatively connected, such as a memory of a hospital information network or a cloud-based storage. The predetermined spatial information can be, for example, distance measurements between different features of the fiducial marker (e.g., distance between corners of the fiducial marker). The predetermined spatial information regarding the fiducial marker may be retrieved from the memory by the image processing system based on information encoded in the fiducial marker. For example, a fiducial marker identification encoded in the fiducial marker may be cross referenced in a fiducial marker database to access the predetermined spatial information associated with that particular fiducial marker.

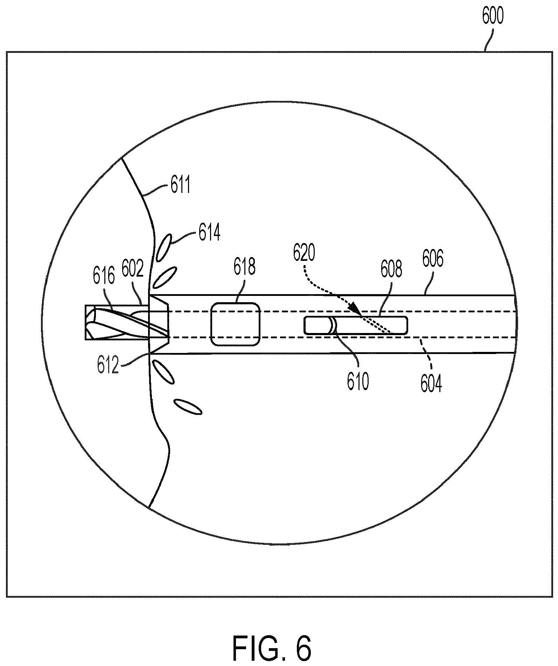

At step 508, whether a hole is drilled and/or is currently being drilled into the tissue is determined. The determination may be performed automatically by one or more components of the system. For example, automatically determining that a hole has been drilled in step 508 may include detection of one or more visual indications in the endoscopic image(s) received at step 502 that are associated with a hole being drilled or having been drilled. The visual indications can be detected, for example, using one or more machine learning models trained to detect such visual indications. Visual indications in the images/videos may include, for example, one or more fiducial markers on the surgical tool (e.g., drill guide shaft), the presence/absence of debris associated with drilling, the presence/absence of a drill bit for a predetermined duration of time, etc., as described in greater detail with respect to FIG. 6.

Alternatively, the image processing system (e.g., image processing system 116 of system 100) may perform one or more steps of method 500 in response to a user command to provide a graphical indication of the hole. The user command can be provided in any suitable fashion, such as via an input associated with a graphical selector in a graphical user interface, an input to a user input device of the image processing system (e.g., a button or a touch screen of the image processing system), a voice command, and/or a press of a button of a camera head of an endoscopic imager. Optionally, the drill used to drill the hole may be communicatively connected to an image processing system and may include a user input device, such as a button, that enables the user to command hole detection by the image processing system.

The determination of the position of the drilled hole described below in step 510 may occur in response to a determination in step 508 that the hole has been drilled. In some embodiments, fiducial marker detection and position/orientation determination, described above with respect to steps 504 and 506, may be performed in response to a determination in step 508 that the hole has been drilled. Alternatively, step 508 may rely upon the position and orientation of the fiducial marker determined at step 506 and, as such, may be performed subsequently to step 506 (as illustrated in FIG. 5).

At step 510, the position in an endoscopic image of a hole drilled in tissue of internal anatomy of a subject by a drill bit disposed within the drill guide is determined based on the position and orientation of the at least one fiducial marker. The position of the hole in the endoscopic image can be determined, for example, by determining the position of the center of the distal end of the drill guide in the endoscopic image, which may be used as the position of the hole (e.g., the position of the entrance to the hole) in the endoscopic image. The position of the center of the distal end of the drill guide can be determined based on the position and orientation of one or more of the fiducial markers identified in the one or more endoscopic images and the predetermined position of the center of the distal end relative to those fiducial markers or relative to one or more portions of those fiducial markers.

Determining the position of the hole can include selecting at least one corner of the fiducial marker or at least one corner of a face on which the fiducial marker is disposed and identifying one or more matching corners in a look-up table, such as table 410 of FIG. 4A, in which one or more corners of one or more fiducial markers disposed on a fiducial ring or one or more corners of one or more faces of the fiducial ring are listed and associated with a corresponding distance to the center of the distal end of the drill guide. The distance to the center of the distal end of the drill guide for the one or more matching corners can be extracted from the look-up table and added and/or subtracted to the position of the one or more corners in the endoscopic image to determine the position of the center of the distal end of the drill guide in the endoscopic image. As described above with respect to FIG. 4A, the table 410 can be determined and generated when the tool is manufactured to ensure that the distances between each corner and the tip are accurately measured and used to populate the table prior to the tool being used in a procedure.

Method 500 can include an optional step 512 of displaying a visualization of the tissue that includes a graphical indication of the determined position of the hole. The visualization can include an endoscopic image with a graphical indication indicating the position of the hole overlayed on the endoscopic image, such as visualization 200 of FIG. 2. The graphical indication can include, for example, a flag or other icon located at the center of the hole. The position and orientation of the fiducial marker determined in step 506 can be used in combination with the predetermined position of the distal end of the drill guide relative to the fiducial marker to determine the shape and position of a graphical indication of the perimeter of a hole relative to an endoscopic image. For example, a circle can be centered at the center of the distal end of the drill guide, scaled in size according to the position of the center of the distal end of the drill guide in a camera-based coordinate system, and rotated in three-dimensional space according to the predetermined orientation of the distal end of the drill guide relative to the fiducial marker and the orientation of the fiducial marker determined in step 506.

The visualization displayed in step 512 may be user modifiable via one or more user input features, such as one or more graphical selectors of a graphical user interface, one or more physical buttons (e.g., on the image processing system 116, on the camera head 108, and/or on the drill 124), a voice command system, etc. For example, the position of the graphical indication of the drilled hole may be user adjustable, which could be useful in scenarios in which the hole position determination was not accurate. Optionally, a user may be able to add and/or delete a graphical indication of a hole, which could be useful in scenarios in which the image processing system failed to generate the graphical indication. The user may be able to select to display and/or hide the graphical indication of the hole.

FIG. 6 illustrates examples of various visual indications associated with hole drilling that may be detected in one or more endoscopic images 600 by the image processing system for automatically determining that a hole has been drilled. One example of a visual indication that a hole 602 is being drilled or has been drilled is the presence of a drill bit 604 in the drill guide 606. The presence of the drill bit 604 in the drill guide 606 may be detected by identifying the drill bit 604 in a window 608 of the drill guide 606. The drill bit 604 may include a marker 610 that may facilitate detection of the drill bit 604 in the drill guide 606. Optionally, the position of the marker 610 in the window 608 may be used as an indication of whether the hole 602 has been completed (e.g., the more distal the marker 610, the deeper the hole 602). A related indication that the hole 602 has been drilled is the disappearance of the drill bit 604 from the window 608, which may indicate that the drill bit 604 has been withdrawn due the hole 602 being completed.

The placement of the drill guide 606 against the tissue 611 is another example of a visual indication that may be used in determining that a hole has been drilled. The position of the distal end 612 of the drill guide 606 may be determined based on the position and orientation of the fiducial marker 618 determined at step 506 and predetermined information about the position of the distal end 612 relative to the fiducial marker 618. The position of the tissue 611 may be determined using, for example, one or more machine learning models trained to identify tissue of interest or features of tissue of interest in endoscopic images.

Another example of a visual indication that a hole has been drilled or is being drilled that may be detected in endoscopic images is the presence of debris 614 created by the drill tip 616 carving out tissue to create the hole 602. Such debris 614 may be detected using a machine learning model trained to detect debris. The presence of the debris 614 may be used as an indication that the hole 602 is being drilled and, optionally, the subsequent absence of the debris 614 may be used as an indication that the hole 602 is no longer being drilled and, thus, is complete.

One or more visual indications associated with hole drilling may be used for purposes other than determining that a hole has been drilled or is being drilled, such as for determining the depth of the hole being drilled. For example, step 510 may include determining a depth of the hole drilled in the tissue based on one or more visual indications of the axial travel of the drill bit when drilling the hole. The axial travel of the drill bit may be determined based on the position of a marker disposed on the drill bit, such as marker 610 of FIG. 6. For example, the image processing system may detect the marker 610 in one or more endoscopic images 600 and determine the location of the marker 610 relative to the distal end 612 of the drill guide 606 based on the position and orientation of the at least one fiducial marker determined in step 506. The image processing system may then determine how far the drill tip 616 has advanced distally of the distal end 612 of the drill guide 606 based on predetermined information associated with the position of the distal end of the drill tip 616 relative to the marker 610 (e.g., the distance from the marker 610 to the distal end of the drill tip 616). The image processing system may store the maximum advancement of the drill tip 616 as the hole depth, updating it over time as new video is captured and analyzed. The hole depth may be displayed to a user (e.g., depth 212 is shown alongside drilled hole graphic 220 in visualization 200 of FIG. 2) to help the user gauge whether to stop drilling or continue drilling. Optionally, the image processing system may determine whether the hole is not deep enough and/or is too deep and may alert the user. For example, the image processing system may compare the depth of the hole to one or more predetermined values or range of values for suitable hole depth (optionally, associated with drill bit size) and may provide an alert that the hole is too shallow when the hole depth is less than the predetermined value(s) or range of values and/or may provide an alert that the hole is too deep when the hole depth is greater than the predetermined value(s) or range of values.

One or more visual indications associated with hole drilling may be used by the image processing system to determine the direction of rotation of the drill bit when drilling the hole. Determining the direction of rotation of the drill bit may be useful, for example, when using drill bits configured to drill different diameters depending on the direction of rotation. Such drill bits may have one or more moveable cutting features that pivot inwardly and outwardly depending on the direction of rotation of the drill bit to drill smaller and larger diameters, respectively. A user may drill into tissue by rotating the drill bit in a first direction to drill a smaller diameter hole and then may rotate the drill in the opposite direction (causing the cutting feature to pivot outwardly) to cut a larger diameter hole at the distal end of the smaller diameter hole (e.g., a counterbore). The image processing system may determine the direction of rotation of the drill bit and may determine based on the direction, whether the smaller diameter and/or larger diameter hole or portions of the hole have been drilled. This determination may be combined with the depth determinations described above to determine how deep the smaller diameter and/or larger diameter hole or portions of the hole are. This information may be displayed to the user, such as shown in visualization 200 of FIG. 2 in which depth 216 is shown alongside a counterbore portion 218 of a drilled hole graphic 220. The direction of rotation of the drill bit may be determined based on the movement of a marker disposed on the drill bit, such as marker 620 of FIG. 6. This marker 620 is non-uniform in the circumferential direction so that it's appearance (e.g., position in a given frame) changes when the drill bit rotates, with the relative change in its appearance between frames corresponding to the direction of rotation.

Determination of the direction of rotation of the drill bit need not be limited to visual indications. For example, the image processing system may be communicatively connected (wired or wireless) to the drill (e.g., image processing system 116 of FIG. 1 may be connected to drill 124) and/or a drill controller and may receive information indicating a direction of rotation of the drill.

Indications that a hole is being drilled or has been drilled that may be used in step 508 need not be limited to visual indications. For example, the activation of the drill (which may be determined, for example, based on a communication from the drill being received at the image processing system) may be used as an indication that the hole is being drilled, and at least in some instances, the subsequent deactivation of the drill may be used as an indication that drilling of the hole has been completed.

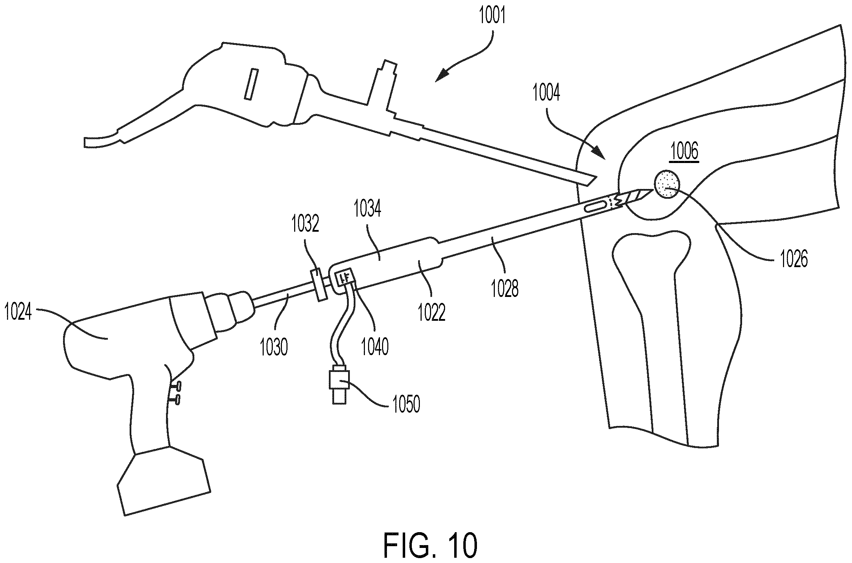

FIG. 10 illustrates another example of a manner of determining that a hole has been drilled. A drill guide 1022 is used for guiding a drill bit 1030 of a drill 1024 to create a hole 1026 in the bone 1006 in a surgical cavity 1004. The drill bit 1030 may include a drill stop 1032, and the proximate portion 1034 of the drill guide 1022 may include a detector 1040 for detecting that the drill stop 1032 has stopped against the proximal portion 1034 of the drill guide 1022. The detector 1040 (illustrated external from the drill guide 1022 for illustration purposes) may be communicatively coupled (e.g., wired via cable 1050 and/or wirelessly) to one or more components of an imaging system that processes images generated by endoscopic imager 1001. For example, the detector 1040 may be communicatively coupled (directly or through one or more intermediate communication devices) to image processing system 116 of FIG. 1. Thus, when the drill bit 1030 is advanced by the surgeon using drill 1024 to drill a hole 1026 into the tissue 1006, the drill stop 1032 coupled to the drill bit 1030 also advances until it abuts the proximate portion 1034 of the drill guide 1022 (thus halting further advancement of the drill bit 1030). The detector 1040 may detect the contact between the drill stop 1032 and the drill guide 1022, and based on the detected contact, may be configured to send signal or message to the imaging system. The imaging system may determine based on the signal or message received from the detector 1040 that the hole 1026 has been drilled in the tissue 1006.

The determination that the hole has been drilled in step 508 may be made based on an amount of time that a given indication is detected. For example, the initial detection of the drill bit 604 in the window 608 may not be used as an indication that the hole 602 has been drilled, but the presence of the drill bit 604 in the window 608 for a threshold amount of time (in this context, "time" can be measured in units of time or in number of video frames) may be used an indication that the hole 602 has been drilled. Relatedly, the presence of debris 614 for a threshold amount of time may be used as an indication that the hole 602 has been drilled.

The determination that the hole has been drilled in step 508 may be based on detection of a combination of different indications. For example, a determination that the hole 602 has been drilled may be based on a combination of detection of the distal end 612 of the drill guide 606 abutting the tissue 611, the presence of the drill bit 604 in the window 608 for a threshold amount of time, and the subsequent absence of the drill bit 604 from the window 608. This is merely an example of the combination of different indications that may be used as a basis for determining that the hole has been drilled. Any combination of the indications described above and/or other indications may be used to determine that a hole has been drilled.

Instead of or in addition to the determination that the hole has been drilled being based on searching for specific indications of hole drilling, the determination may be made using a machine learning model trained to detect a hole drilling operation, which is often referred to as online action detection. The machine learning model may be trained to detect the start and end of specific action instances in a video. For example, the action to be detected may be defined as "drilling a hole in tissue" (e.g., in the context of a specific type of surgery, such as arthroscopic surgery), and a temporal modelling machine learning model such as Long Short Term Transformer (LSTR) may be trained to detect such an action. Given a live streaming video, LSTR sequentially identifies the actions happening in each incoming frame, without future context. The advantage of this approach compared to specifying and detecting indirect events correlated with hole drilling (e.g., presence of debris, visibility of a marker) is that it can automatically account for many more sub-events accompanying hole drilling that occur in tandem, hence leading to increased detection accuracy. The machine learning model may be trained on labeled training data that includes endoscopic videos of holes being drilled to completion such that the machine learning model learns to identify hole drilling and completion.

To improve the accuracy of detecting the indications that a hole is being or has been drilled and/or to reduce computational intensity, one or more techniques can be used to narrow the region of the endoscopic image that is searched for indications of hole drilling. For example, where a window of the drill guide is monitored to determine whether the drill bit is present in the window, the position of the window in the endoscopic image may first be determined and the search for the presence of the drill bit in the window may be limited to region of the endoscopic image associated with the window. The determination of the fiducial marker position and orientation from step 506 may be used to determine the position of the window. For example, with reference to FIG. 6, the position of the window 608 relative to the fiducial marker 618 may be predetermined and this information may be obtained from a database by the image processing system and used to limit the area of the endoscopic image that will be analyzed for the presence of the drill bit. Optionally, a machine learning model may be used to identify the window in the endoscopic image based on analyzing the region of the endoscopic image associated with the window or based on analyzing the entire endoscopic image.

The search for a particular indication that a hole is being drilled or has been drilled may be conditioned on detecting one or more other indications. In this way, the image processing system need not analyze the endoscopic image for all indications at all times, which can improve accuracy and/or reduce computational intensity. For example, the image processing system may first search for the presence of the drill guide within the field of view (e.g., detected by identifying the fiducial marker). Once the presence of the drill guide is detected, the image processing system may begin searching for the presence of the drill bit in the drill guide. Once the drill bit is detected in the drill guide, the image processing system may begin searching for debris associated with drilling.

As explained above, method 500 may include the step 512 of displaying a visualization of the tissue that includes a graphical indication of the position of the hole in one or more endoscopic images. The one or more endoscopic images may be frames of a video feed and the visualization may include displaying the graphical indication as an overlay on the video. Since the endoscopic imager may move relative to the imaged tissue over time, the image processing system may track such movement and may relocate the graphical indication in the visualization such that the graphical indication continues to be positioned at the correct position relative to the drilled hole.

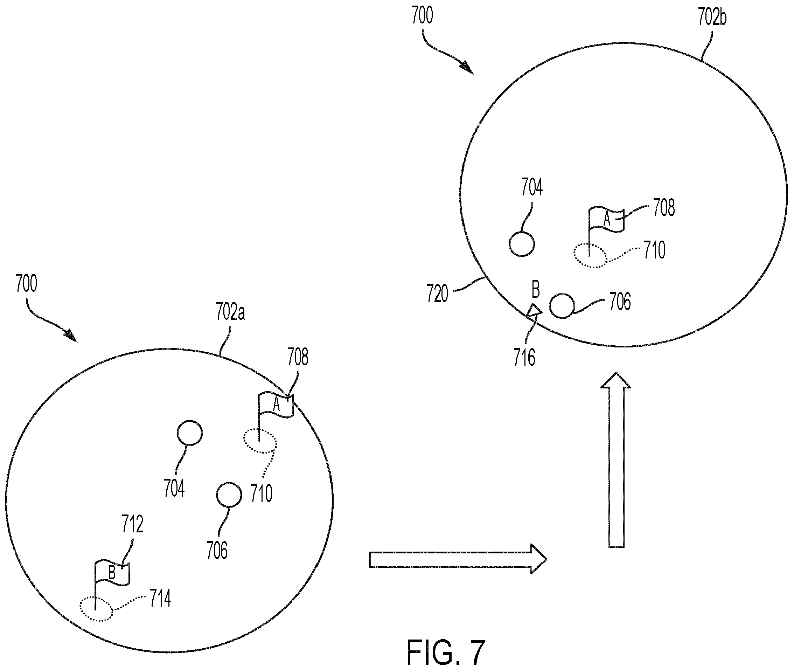

FIG. 7 illustrates an exemplary visualization of tissue of interest in which one or more graphical indications of one or more holes drilled in the tissue are relocated in the visualization based on movement of the endoscopic imager such that the graphical indication(s) continues to accurately indicate the position of the hole(s) relative to the displayed scene. FIG. 7 illustrates two separate instantiations of a visualization 700, each including a different frame 702a and 702b of an endoscopic video. Frame 702a was taken earlier in time than frame 702b. Frames 702a and 702b can each capture multiple features of interest 704 and 706 of tissue within the field of view, which as described further below, can be identified for determining camera movement. Visualization 700 can include a flag 708 that indicates a hole 710 drilled in the tissue. The position of the flag 708 in the visualization 700 for frame 702a may be determined according to method 500 of FIG. 5. Thus, while features of interest 704 and 706 represent parts of the anatomy that are visible within the frame 702a, flag 708 is a graphical feature overlayed on the frame 702a. One or more additional flags 712 may be displayed for additional holes 714 drilled in the tissue.

If the endoscopic imager used to generate frame 702a remains stationary, then features of interest 704 and 706, as well as flag 708, can maintain their position within the visualization 700. However, if the endoscopic imager moves during the procedure, then the position of features of interest 704 and 706, as well as the hole 710 indicated by flag 708, will move within the visualization, based on the direction and distance that the endoscopic imager moves. For instance, as shown in FIG. 7, frame 702b can represent a frame that would appear if the endoscopic imager is moved to the right and up. In such an example, the position of features of interest 704 and 706 will appear further down and to the left in frame 702b than in frame 702a. Likewise, flag 708 of frame 702 will be moved in the visualization 700 further down and to the left in the visualization 700.

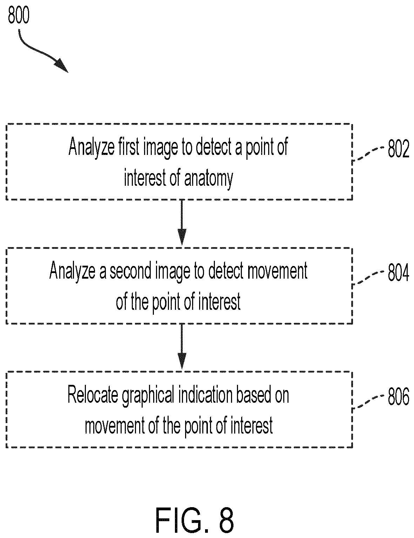

FIG. 8 illustrates an exemplary method 800 for repositioning a graphical indication of a hole drilled in tissue in a visualization according to movement of an endoscopic imager. Method 800 can be performed in combination with one or more steps of method 500 of FIG. 5. For example, method 800 can be performed for a visualization is displayed in step 512 based on frames of an endoscopic video received in step 502. Method 800 can be performed by an image processing system, such as image processing system 116 of FIG. 1.

At step 802, at least one point of interest of the anatomy captured in a received frame (or multiple frames) of endoscopic video can be detected. The points of interest can be identified using one or more machine learning models configured to detect points of interest in images for use in computer vision applications such as the Greedily Learned Accurate Match Points (GLAM) keypoint detector, the Self-Supervised Interest Point Detector (Superpoint), Magicpoint, Unsuperpoint, or other machine learning models configured to learn correspondences in images with known homographic transformations. The machine learning model or models used to detect points of interests (i.e., keypoints) in an image can be configured to extract one or more stable points in an image. Stable points can be used to track the motion of an endoscopic imager such as to determine the rotation and/or translation of the endoscopic imager, based on the movement of those points in a video feed and/or a subsequent frame of the image. The frame within which the at least one point of interest is detected may be displayed as part of a visualization that provides a graphical indication of a hole drilled in tissue according to method 500, such as frame 702a of visualization 700 of FIG. 7.

At step 804, a frame captured after the frame used in step 802 was captured is received and used to determine whether, and the extent to which, the endoscopic imager has moved during the time between when the first frame was captured and when the second frame was captured. The at least one point of interest detected at step 802 can be also detected in step 804 using one or more of the machine learning models described above with respect to step 802. If the camera has moved between capture of the first frame and capture of the second frame, then the points of interest located in the first frame will have moved to a different position in the image frame based on the extent and direction of the motion. The movement of the interest points can be used to determine where to place the graphical indication of the hole relative to the second frame so that the graphical indication appears stuck to the drilled hole and moves according to the motion of the endoscopic imager so that the graphical indication points to hole despite the motion of the camera.

The distance and direction of the movement of the endoscopic imager can be calculated using homography, a computer vision technique that can be used to produce a motion matrix that indicates how much each pixel in the image has moved from a previous image using one or more homographic transformations. The frames of steps 802 and 804 can be matched using a k-nearest neighbors (KNN) algorithm with filtering that can be used to match the at least one point of interest identified in the frames. From the matched correspondences, the estimation of motion can be performed using homography.

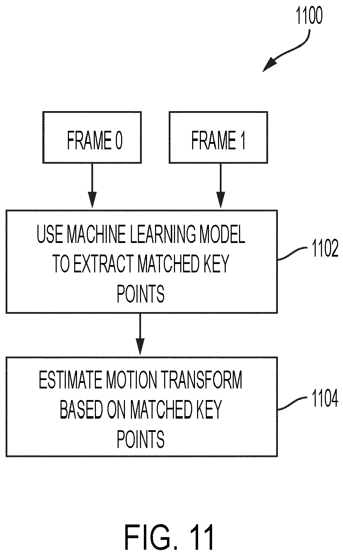

Points of interest (also referred to as key points) determination according to steps 802 and 804 need not be performed in discrete steps. Rather, a machine learning model may be used that analyzes a received pair of frames together to extract matched key points between the frames. The matched key points can then be used to estimate motion between the frames. FIG. 11 is a flow diagram of an exemplary method 1100 that can be performed by the image processing system to estimate motion of an endoscopic imager between frames, which can be used in place of steps 802 and 804 of method 1100. At step 1102, a machine learning model processes a received pair of frames-frame0 and frame1-and outputs a set of matched pairs of key points and their relative motions in three dimensions. The machine learning model used in step 1102 can be a transformer-based matching model trained using frames captured by a camera with viewpoint changes and/or illumination changes and camera positional information corresponding to the position of the camera for each frame. Examples of suitable machine learning models include Local Feature Transformer ("LoFTR"), MatchFormer, QuadTreeAttention, and TransMatcher. At step 1104, the matched key points are used to generate an estimate of motion of the endoscopic imager from frame0 to frame1. In step 1104, if the differences in relative locations of the matched key points are sufficiently large, then essential matrix techniques can be used to generate an essential matrix that relates corresponding key points in frame0 and frame1. The essential matrix can be used to derive an estimate of the translation and rotation (six degree of freedom motion) of the endoscopic imager from frame0 to frame1. If at least one of frame0 and frame1 includes a fiducial marker, then the characteristics of fiducial marker can be used to scale the essential matrix to generate a scaled estimate of motion (e.g., in millimeters). Thus, method 1100 can be used to generate an estimate of six-dimensional motion of the endoscopic imager from frame0 to frame1. This motion estimate can be used in step 806 of method 800 to transform the position of the drilled hole from its position in frame0 to its position in frame1 and relocate the graphical indication accordingly.

Other machine learning frameworks (individual machine learning models or combinations of multiple machine learning models) that do not rely upon extracting matched key points may be used for determining motion from pairs of frames. An example of such a machine learning framework is a unified self-supervised machine learning framework that estimates monocular depth and ego-motion simultaneously, such as described in a paper titled Self-Supervised Monocular Depth and Ego-Motion Estimation in Endoscopy: Appearance Flow to the Rescue, by Shuwei Shao et al., arXiv:2112.08122v1 [cs.CV] 15 Dec 2021 (available at https://arxiv.org/pdf/2112.08122.pdf), the entire contents of which are incorporated by reference herein. This machine learning framework includes a structure module, a motion module, an appearance module, and a correspondence module to accurately reconstruct the appearance and calibrate the brightness of the frames. The structure module is a mono-depth estimator that converts a target frame into a dense depth map. The motion module functions as a six degree-of-freedom ego-motion estimator, which takes two adjacent frames as input and outputs a relative pose. The appearance module is used to predict appearance flow and align the brightness condition through a brightness calibration procedure. The correspondence module performs an automatic registration step. This machine learning framework can be trained on unlabeled endoscopic (e.g., arthroscopic) video datasets. The trained machine learning framework can then be used to estimate three-dimensional inter-frame deformation and displacement, without relying on key points or a labeled dataset.

It may not be possible to estimate motion between two frames directly, such as when the two frames are too dissimilar to match key points due to excessive motion of the endoscopic imager between the two frames. However, motion between the two frames can still be estimated by combining estimates of motion generated from one or more intermediate frames. For example, where the drilled hole was initially determined based on frame Fₒ, the frame of interest is Fₙ, and a frame Fₙ₋₁ was captured after frame Fₒ and before frame Fₙ, the motion between Fₒ and Fₙ can be determined by combining an estimate of motion of the endoscopic imager from frame Fₒ to frame Fₙ₋₁ with an estimate of motion of the endoscopic imager from frame Fₙ₋₁ to frame Fₙ. This can be done for any number of frames captured between frame Fₒ and frame Fₙ (e.g., Fₒ→Fₒ₊₁ + Fₒ₊₁→Fₒ₊₂ + ... + Fₙ₋₂→Fₙ₋₁ + Fₙ₋₁→Fₙ). As such, method 1100 can be performed for multiple sets of frames to generate multiple step-wise motion estimates that can be combined to determine motion from the frame in which a drilled hole was determined to a frame for which the position of the drilled hole is to be determined. This motion can then be applied to the position of one or more points in frame Fₙ to transform them to the positions they have in frame Fₒ. Thus, where the position of the drilled hole is known in frame Fₒ, its position in (or relative to) frame Fₙ can be determined by transforming (e.g., via matrix multiplication) its position in frame Fₒ using the sum of the motion estimates generated by method 1100.

It may not be necessary to use all frames captured between Fₒ and frame Fₙ to determine the motion of the endoscopic camera. For example, where Fₒ and frame Fₒ₊₁ are sufficiently similar due to little or no motion of the endoscopic imager, frame Fₒ₊₁ does not need to be used to generate motion estimates. Rather, the frames used to estimate motion between frame Fₒ and frame Fₙ can be a select set of key frames that have been selected based on their suitability for estimating motion. For example, where Fₒ₊₂ has been selected as a key frame, method 1100 can be applied to frames Fₒ and Fₒ₊₂ to compute the motion directly from frame Fₒ to frame Fₒ₊₂.

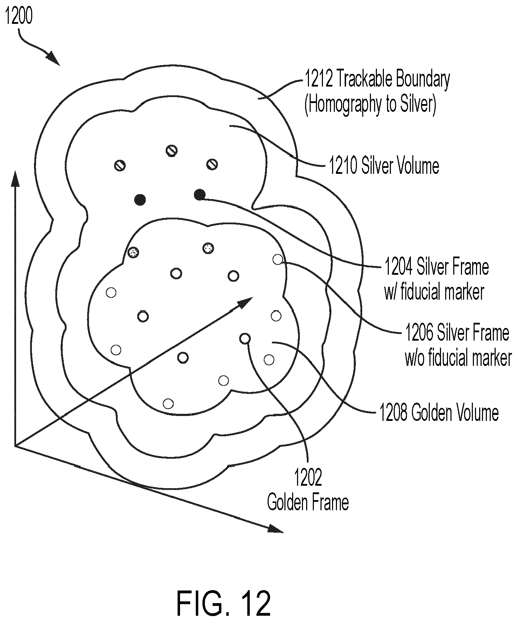

Key frames can be selected over time as frames are being captured by comparing newly captured frames to one or more previously selected key frames to determine whether there has been sufficient motion to warrant the selection of a new key frame. Over time, a key frame "cloud" can be generated that spans (with a tunable degree of density) the six degree of freedom space of endoscopic imager position and orientation. The key frame cloud can include a set of key frames stored in memory along with their corresponding six degree of freedom positions and orientations. A conceptual representation of a simplified key frame cloud representing only three degrees of freedom is illustrated in FIG. 12. Each point in the key frame cloud 1200 corresponds to a different key frame, with its location being associated with the position and orientation of the endoscopic imager (e.g., the position and orientation of the distal end of the endoscopic imager) at the time the key frame was captured. As such, each point in the key frame cloud 1200 represents a somewhat different scene from the other points in the key frame cloud 1200.

Key frame cloud 1200 may include several different types of key frames. A "golden" frame 1202 is a frame for which the position of a drilled hole (or any other point of interest) has been determined, such as via steps 402 to 408 of method 400 of FIG. 4 or steps 802 to 810 of method 800 of FIG. 8. There can be multiple golden frames 1202 if, for example, positions of multiple holes to be drilled have been determined. The golden volume 1208 is the portion of the six degree of freedom space for which motion can be determined from a golden frame 1202. In other words, a given frame within the golden volume 1208 and at least one golden frame 1202 can be analyzed (e.g., using method 1100) to determine the motion between the given frame and the golden frame 1202. Points outside the golden volume 1208 are too far from a nearest golden frame 1202 for motion to be determined using a golden frame 1202 directly.

The silver frames 1204 and 1206 are key frames whose position and orientation have been determined based on a golden frame 1202 or another silver frame 1204 or 1206, such as by using method 1100. Each silver frame 1204 and 1206 is sufficiently "far" from the golden frames 1202 and other silver frames 1204 and 1206 to warrant being included in the key frame cloud 1200 but not so far that the motion estimate back to a golden frame 1204 or other key frame 1204 and 1206 is unreliable or not computable. The density of the key frames (i.e., the minimum distance between key frames in six-dimensional space) can be set based on memory, computational performance, and/or motion accuracy requirements, with a sparser key frame cloud requiring less memory and less processing time than a denser key frame cloud at the potential expense of motion estimation accuracy. There may be at least two types of silver frames. Silver frames 1204 include a fiducial marker and silver frames 1206 do not include a fiducial marker. A fiducial marker in a frame can be used to "scale" the motion estimate generated in method 1100. As such, a silver frame 1206 that does not have a fiducial marker is sufficiently close to either a golden frame 1202 or a silver frame 1204 that does include a fiducial marker to enable a scaled motion estimate to be generated for the silver frame 1206. The silver volume 1210 is the portion of the six degree of freedom space outside of the golden volume 1208 for which motion can be determined from a silver frame 1206 that includes a fiducial marker. The trackable boundary 1212 represents the limit of six degree of freedom space for which motion can be computed from any silver frame 1204 or 1206 using homography, such as discussed above with respect to step 804 of method 800.

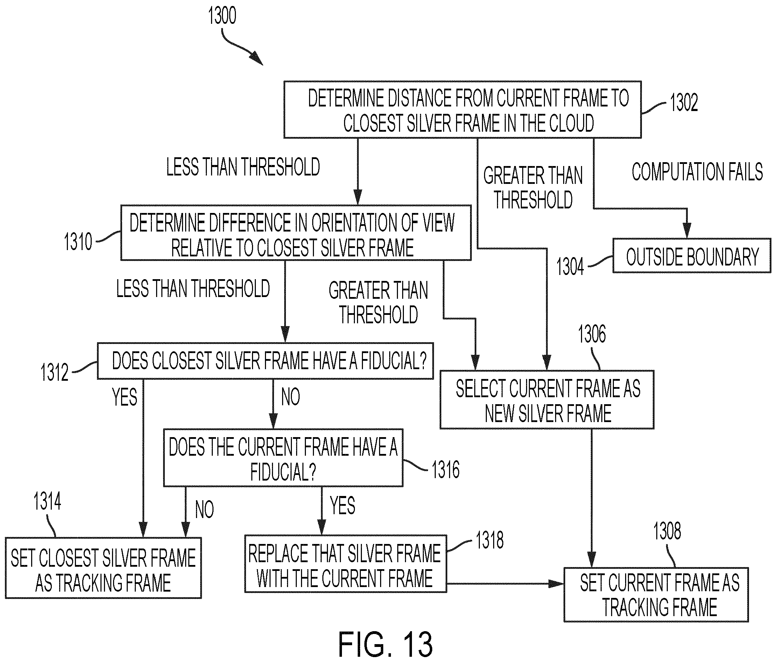

FIG. 13 is a flow diagram of an exemplary method 1300 for building a key frame cloud, such as key frame cloud 1200 of FIG. 12. Method 1300 can be performed by the image processing system periodically as frames are being captured (e.g., on each frame, every other frame, every third frame, etc.) and received by the image processing system. At step 1302, the distance from a current frame to a closest key frame in a key frame cloud is determined. The closest key frame can be a silver frame, as illustrated in FIG. 13, or can be a golden frame. The location of the current frame can be determined by estimating motion from a previous frame for which location has been determined (e.g., a tracking frame, as described below) to the current frame. This can be done by using homography and/or method 1100 to determine motion of key points and then computing an average motion across the key points of the frame. The key frame cloud is searched for a key frame that is closest in location (e.g., three-dimensional location) to the current frame's location and the distance between them is calculated. If for some reason this determination fails, such as if the current frame is too dissimilar from a previous frame due to excessive motion of the endoscopic camera (or, perhaps, excessive motion of the subject), then the current frame is determined to be outside the trackable boundary of the point cloud at step 1304 and the process ends (a warning may be provided to the user indicating excessive camera and/or subject motion).

If the distance between the current frame and closest key frame, as determined in step 1302, is greater than a threshold distance, then the current frame is selected as a new silver frame for the key frame cloud at step 1306. The threshold distance can be a physical-space distance, such as a certain number of millimeters. The current frame is then set as the tracking frame at step 1308. The tracking frame can be used for tracking motion of future frames.

If the distance between the current frame and closest key frame, as determined in step 1302, is less than the threshold distance, then a difference in orientation of the view of the current frame relative to the view of the closest key frame is determined in step 1310. If the difference in orientation is greater than a threshold (e.g., a threshold value in degrees), then method 1300 proceeds to step 1306. If the difference in orientation is less than the threshold, then at step 1312, a determination is made whether the closest key frame has a fiducial. If it does, then at step 1314, the closest key frame is set as the tracking frame. If the closest key frame does not have a fiducial, then at step 1316, a determination is made of whether the current frame has a fiducial. If it does not, then method 1300 proceeds to step 1314. If the current frame does have a fiducial, then at step 1318, the current key frame is replaced in the key frame cloud with the current frame, and the method continues to step 1308.

Method 1300 need not be performed on frames that do not have much motion relative to a previous frame. As such, method 1300 may only be performed if the motion between a current frame and a previous frame is sufficiently large. The motion between a current frame and a previous frame can be estimated using a two-dimensional motion technique, such as homography, and if the motion estimate is larger than a threshold, then method 1300 can be performed. The threshold can be, for example, a threshold number of pixels or a threshold percentage of the scope circle radius. If the estimate is below the threshold, then the two-dimensional motion estimate can be used to update the position of a drilled hole (or other feature of interest) for the current frame. The previous frame that the current frame is compared to can be, for example, a tracking frame determined in a previous iteration of method 1300. The motion between a current frame and a golden frame used to determine the location of a drilled hole (or other feature of interest) in the current frame can be determined by calculating motion from the current frame to the tracking frame and from the tracking frame back through key frames of the key frame cloud to the golden frame in a step-wise fashion. The step-wise motions can be accumulated (e.g., by adding them together).

At step 806, the amount of motion estimated at step 804 is used to move the graphical indication within the visualization in accordance with the estimated motion of the endoscopic imager so that the at least one graphical indication continues to point to the hole drilled in the tissue. If the amount of motion determined between the first captured frame and the second captured frame is such that the hole is no longer captured in the frame (and, as a result, the graphical indication cannot be placed in the field of view), then a marker can be placed at the edge of the field of view in the visualization indicating a general direction of the hole. An example of this is shown in FIG. 7 in which hole 714 has moved out of the field of view (i.e., is not captured in frame 702b) and a marker 716 is displayed at the edge of the field of view 720 to indicate where the hole 714 is located relative to the currently displayed field of view.

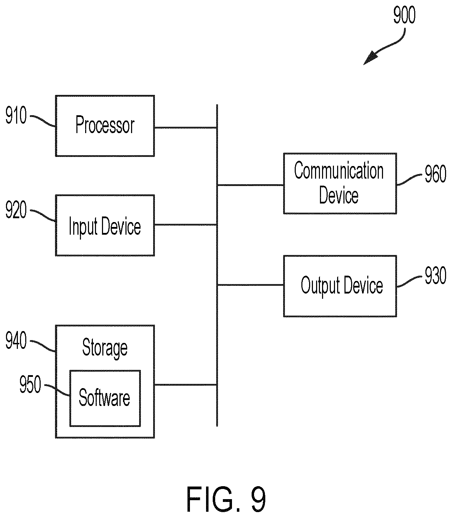

FIG. 9 illustrates an example of a computing system 900 that can be used for one or more components of system 100 of FIG. 1, such as one or more of camera head 108, camera control unit 112, and image processing system 116. System 900 can be a computer connected to a network, such as one or more networks of hospital, including a local area network within a room of a medical facility and a network linking different portions of the medical facility. System 900 can be a client or a server. System 900 can be any suitable type of processor-based system, such as a personal computer, workstation, server, handheld computing device (portable electronic device) such as a phone or tablet, or dedicated device. System 900 can include, for example, one or more of input device 920, output device 930, one or more processors 910, storage 940, and communication device 960. Input device 920 and output device 930 can generally correspond to those described above and can either be connectable or integrated with the computer.

Input device 920 can be any suitable device that provides input, such as a touch screen, keyboard or keypad, mouse, gesture recognition component of a virtual/augmented reality system, or voice-recognition device. Output device 930 can be or include any suitable device that provides output, such as a display, touch screen, haptics device, virtual/augmented reality display, or speaker.

Storage 940 can be any suitable device that provides storage, such as an electrical, magnetic, or optical memory including a RAM, cache, hard drive, removable storage disk, or other non-transitory computer readable medium. Communication device 960 can include any suitable device capable of transmitting and receiving signals over a network, such as a network interface chip or device. The components of the computing system 900 can be connected in any suitable manner, such as via a physical bus or wirelessly.

Processor(s) 910 can be any suitable processor or combination of processors, including any of, or any combination of, a central processing unit (CPU), field programmable gate array (FPGA), and application-specific integrated circuit (ASIC). Software 950, which can be stored in storage 940 and executed by one or more processors 910, can include, for example, the programming that embodies the functionality or portions of the functionality of the present disclosure (e.g., as embodied in the devices as described above), such as programming for performing one or more steps of method 500 of FIG. 5 and/or method 800 of FIG. 8

Software 950 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 940, that can contain or store programming for use by or in connection with an instruction execution system, apparatus, or device.

Software 950 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport computer readable medium can include, but is not limited to, an electronic, magnetic, optical, electromagnetic, or infrared wired or wireless propagation medium.

System 900 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

System 900 can implement any operating system suitable for operating on the network. Software 950 can be written in any suitable programming language, such as C, C++, Java, or Python. In various embodiments, application software embodying the functionality of the present disclosure can be deployed in different configurations, such as in a client/server arrangement or through a Web browser as a Web-based application or Web service, for example.

The disclosure will now further describe the following numbered embodiments. It will be appreciated that some or all of the embodiments summarize aspects of the disclosure as provided in the detailed description and the figures. Accordingly, the embodiments are also to be read in connection with the preceding paragraphs and the appended figures, and do not limit the disclosure. The features and preferences as described hereinabove apply also to the following embodiments.
Embodiment 1: A drill guide for use in endoscopic surgical procedures, the drill guide configured to guide a drill bit to drill a hole in tissue of internal anatomy of a patient, the drill guide comprising:
   a shaft configured to receive the drill bit, wherein a distal end of the shaft is configured to interface with the tissue; and
   at least one fiducial marker positioned proximate the distal end of the shaft and configured to provide position information for the hole in an endoscopic image captured by an endoscopic imaging device.
Embodiment 2: The drill guide of embodiment 1, wherein the shaft comprises a window for viewing the drill bit when the drill bit is received in the shaft.
Embodiment 3: The drill guide of embodiment 2, wherein the window is configured for viewing a marker disposed on the drill bit when the drill bit is drilling the hole in the tissue.
Embodiment 4: The drill guide of any one of embodiments 1-3, wherein the at least one fiducial marker comprises at least one ArUco marker.
Embodiment 5: The drill guide of any one of embodiments 1-4, wherein the at least one fiducial marker comprises a plurality of fiducial markers.
Embodiment 6: The drill guide of embodiment 5, wherein each fiducial marker of the plurality of fiducial markers is disposed on a different face of a fiducial ring.
Embodiment 7: The drill guide of embodiment 6, wherein each fiducial marker of the plurality of fiducial markers is configured to identify which face of the fiducial ring on which the fiducial marker is disposed.
Embodiment 8: The drill guide of any one of embodiments 6-7, wherein the fiducial ring is coaxial with the shaft.
Embodiment 9: The drill guide of any one of embodiments 5-8, wherein each fiducial marker of the plurality of fiducial markers comprises identifying information that uniquely identifies the respective fiducial marker relative to the other fiducial markers of the plurality of fiducial markers.
Embodiment 10: A method for determining a position of a hole drilled in tissue of internal anatomy of a patient by a drill bit disposed within a drill guide, the method comprising, at a computing system:
   receiving at least one endoscopic image that captures at least a distal portion of a shaft of the drill guide;
   identifying in the at least one endoscopic image at least one fiducial marker disposed on the distal portion of the shaft of the drill guide;
   determining a position and orientation of the at least one fiducial marker;
   determining that the drill bit drilled the hole in the tissue; and
   determining the position of the hole based on the position and orientation of the at least one fiducial marker.
Embodiment 11: The method of embodiment 10, further comprising displaying a visualization of the tissue that comprises a marker indicating the position of the hole. Embodiment 12: The method of embodiment 11, wherein displaying the visualization comprises overlaying the marker on an endoscopic video and updating the position of the marker based on changes in the field of view of the endoscopic video.
Embodiment 13: The method of any one of embodiments 11-12, further comprising modifying the display of the visualization of the tissue based on at least one user input.
Embodiment 14: The method of any one of embodiments 10-13, wherein determining that the drill bit drilled the hole in the tissue comprises determining from the at least one endoscopic image that the drill bit drilled the hole in the tissue.
Embodiment 15: The method of embodiment 14, wherein determining from the at least one endoscopic image that the drill bit drilled the hole in the tissue comprises determining that the drill bit is visible in a window of the shaft.
Embodiment 16: The method of embodiment 15, wherein determining from the at least one endoscopic image that the drill bit drilled the hole in the tissue comprises determining that the drill bit has been visible in the window of the shaft for at least a predetermined period.
Embodiment 17: The method of embodiment 16, wherein the predetermined period is a predetermined time period or a predetermined number of endoscopic images.
Embodiment 18: The method of any one of embodiments 15-17, further comprising locating the window of the shaft in the at least one image based on the position and orientation of the at least one fiducial marker.
Embodiment 19: The method of any one of embodiments 14-18, wherein determining from the at least one endoscopic image that the drill bit drilled the hole in the tissue comprises determining that a marker on the drill bit is visible in a window of the shaft.
Embodiment 20: The method of any one of embodiments 14-19, wherein determining from the at least one endoscopic image that the drill bit drilled the hole in the tissue comprises detecting debris created by the drill bit drilling the hole in the at least one image.
Embodiment 21: The method of any one of embodiments 10-20, wherein identifying the at least one fiducial marker disposed on the shaft of the drill guide comprises applying one or more machine learning models to the received endoscopic image to segment the drill guide in the at least one endoscopic image.
Embodiment 22: The method of any one of embodiments 10-21, wherein identifying the at least one fiducial marker comprises:
   identifying one or more visual patterns of the at least one fiducial marker; and
   matching the one or more visual patterns of the at least one fiducial marker to at least one fiducial marker pattern in a predefined list of fiducial marker patterns.
Embodiment 23: The method of any one of embodiments 10-22, wherein the at least one fiducial marker is disposed on a face of a fiducial ring.
Embodiment 24: The method of embodiment 23, wherein determining the position of the hole comprises:
   selecting a corner of the face corresponding to the at least one fiducial marker;
   identifying a position of the selected corner with respect to the drill guide based on the determined position and orientation of the at least one fiducial marker; and
   determining a three-dimensional distance from the selected corner to a center of a distal end of the drill guide based on the identified position of the selected corner with respect to the drill guide.
Embodiment 25: The method of any one of embodiment s 23-24, wherein determining the three-dimensional distance from the selected corner to the hole comprises:
   identifying a matching corner corresponding to the selected corner, the matching corner stored in a look-up table comprising a list of fiducial markers and corresponding corners, wherein each entry pertaining to a corner in the look-up table comprises a predetermined three-dimensional distance between the corner and a position of the center of the distal end of the drill guide; and
   extracting the predetermined three-dimensional distance corresponding to the identified matching corner corresponding to the selected corner in the look-up table, wherein the position of the center of the distal end of the drill guide is determined based on the predetermined three-dimensional distance.
Embodiment 26: A system for determining a position of a hole drilled in tissue of internal anatomy of a patient by a drill bit disposed within a drill guide, the system comprising one or more processors, memory, and one or more programs stored in the memory and including instructions for execution by the one or more processors for:
   receiving at least one endoscopic image that captures at least a distal portion of a shaft of the drill guide;
   identifying in the at least one endoscopic image at least one fiducial marker disposed on the shaft of the drill guide;
   determining a position and orientation of the at least one fiducial marker;
   determining that the drill bit drilled the hole in the tissue; and
   determining the position of the hole based on the position and orientation of the at least one fiducial marker.
Embodiment 27: The system of embodiment 26, wherein the one or more programs include instructions for displaying a visualization of the tissue that comprises a marker indicating the position of the hole.
Embodiment 28: The system of embodiment 27, wherein displaying the visualization comprises overlaying the marker on an endoscopic video and updating the position of the marker based on changes in the field of view of the endoscopic video.
Embodiment 29: The system of any one of embodiments 27-28, wherein the one or more programs include instructions for modifying the display of the visualization of the tissue based on at least one user input.
Embodiment 30: The system of any one of embodiments 26-29, wherein determining that the drill bit drilled the hole in the tissue comprises determining from the at least one endoscopic image that the drill bit drilled the hole in the tissue.
Embodiment 31: The system of embodiment 30, wherein determining from the at least one endoscopic image that the drill bit drilled the hole in the tissue comprises determining that the drill bit is visible in a window of the shaft.
Embodiment 32: The system of embodiment 31, wherein determining from the at least one endoscopic image that the drill bit drilled the hole in the tissue comprises determining that the drill bit has been visible in the window of the shaft for at least a predetermined period.
Embodiment 33: The system of embodiment 32, wherein the predetermined period is a predetermined time period or a predetermined number of endoscopic images.
Embodiment 34: The system of any one of embodiments 31-33, wherein the one or more programs include instructions for locating the window of the shaft in the at least one image based on the position and orientation of the at least one fiducial marker.
Embodiment 35: The system of any one of embodiments 30-34, wherein determining from the at least one endoscopic image that the drill bit drilled the hole in the tissue comprises determining that a marker on the drill bit is visible in a window of the shaft.
Embodiment 36: The system of any one of embodiments 30-35, wherein determining from the at least one endoscopic image that the drill bit drilled the hole in the tissue comprises detecting debris created by the drill bit drilling the hole in the at least one image.
Embodiment 37: The system of any one of embodiments 26-36, wherein identifying the at least one fiducial marker disposed on the shaft of the drill guide comprises applying one or more machine learning models to the received endoscopic image to segment the drill guide in the at least one endoscopic image.
Embodiment 38: The system of any one of embodiments 26-37, wherein identifying the at least one fiducial marker comprises:
   identifying one or more visual patterns of the at least one fiducial marker; and
   matching the one or more visual patterns of the at least one fiducial marker to at least one fiducial marker pattern in a predefined list of fiducial marker patterns.
Embodiment 39: The system of any one of embodiments 26-38, wherein the at least one fiducial marker is disposed on a face of a fiducial ring.
Embodiment 40: The system of embodiment 39, wherein determining the position of the hole comprises:
   selecting a corner of the face corresponding to the at least one fiducial marker;
   identifying a position of the selected corner with respect to the drill guide based on the determined position and orientation of the at least one fiducial marker; and
   determining a three-dimensional distance from the selected corner to a center of a distal end of the drill guide based on the identified position of the selected corner with respect to the drill guide.
Embodiment 41: The method of any one of embodiments 39-40, wherein determining the three-dimensional distance from the selected corner to the hole comprises:
   identifying a matching corner corresponding to the selected corner, the matching corner stored in a look-up table comprising a list of fiducial markers and corresponding corners, wherein each entry pertaining to a corner in the look-up table comprises a predetermined three-dimensional distance between the corner and a position of the center of the distal end of the drill guide; and
   extracting the predetermined three-dimensional distance corresponding to the identified matching corner corresponding to the selected corner in the look-up table, wherein the position of the center of the distal end of the drill guide is determined based on the predetermined three-dimensional distance.

The foregoing description, for the purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the techniques and their practical applications. Others skilled in the art are thereby enabled to best utilize the techniques and various embodiments with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of the patents and publications referred to in this application are hereby incorporated herein by reference.

## Claims

1. A drill guide for use in endoscopic surgical procedures, the drill guide configured to guide a drill bit to drill a hole in tissue of internal anatomy of a patient, the drill guide comprising:
a shaft configured to receive the drill bit, wherein a distal end of the shaft is configured to interface with the tissue; and
at least one fiducial marker positioned proximate the distal end of the shaft and configured to provide position information for the hole in an endoscopic image captured by an endoscopic imaging device.

2. The drill guide of claim 1, wherein the shaft comprises a window for viewing the drill bit when the drill bit is received in the shaft, wherein optionally the window is configured for viewing a marker disposed on the drill bit when the drill bit is drilling the hole in the tissue.

3. The drill guide of claim 1 or 2, wherein the at least one fiducial marker comprises at least one ArUco marker.

4. The drill guide of any one of claims 1-3, wherein the at least one fiducial marker comprises a plurality of fiducial markers.

5. The drill guide of claim 4, wherein each fiducial marker of the plurality of fiducial markers is disposed on a different face of a fiducial ring, wherein optionally each fiducial marker of the plurality of fiducial markers is configured to identify which face of the fiducial ring on which the fiducial marker is disposed, and wherein optionally the fiducial ring is coaxial with the shaft.

6. The drill guide of claim 4 or 5, wherein each fiducial marker of the plurality of fiducial markers comprises identifying information that uniquely identifies the respective fiducial marker relative to the other fiducial markers of the plurality of fiducial markers.

7. A system for determining a position of a hole drilled in tissue of internal anatomy of a patient by a drill bit disposed within a drill guide for instance according to any of claims 1-6, the system comprising one or more processors, memory, and one or more programs stored in the memory and including instructions for execution by the one or more processors for:
receiving at least one endoscopic image that captures at least a distal portion of a shaft of the drill guide;
identifying in the at least one endoscopic image at least one fiducial marker disposed on the shaft of the drill guide;
determining a position and orientation of the at least one fiducial marker;
determining that the drill bit drilled the hole in the tissue; and
determining the position of the hole based on the position and orientation of the at least one fiducial marker.

8. The system of claim 7, wherein the one or more programs include instructions for displaying a visualization of the tissue that comprises a marker indicating the position of the hole, wherein displaying the visualization optionally comprises overlaying the marker on an endoscopic video and updating the position of the marker based on changes in the field of view of the endoscopic video, and/or wherein the one or more programs optionally include instructions for modifying the display of the visualization of the tissue based on at least one user input.

9. The system of claim 7 or 8, wherein determining that the drill bit drilled the hole in the tissue comprises determining from the at least one endoscopic image that the drill bit drilled the hole in the tissue, wherein optionally a) determining from the at least one endoscopic image that the drill bit drilled the hole in the tissue comprises determining that the drill bit is visible in a window of the shaft, wherein further optionally i) determining from the at least one endoscopic image that the drill bit drilled the hole in the tissue comprises determining that the drill bit has been visible in the window of the shaft for at least a predetermined period, such as a predetermined time period or a predetermined number of endoscopic images, and/or ii) the one or more programs include instructions for locating the window of the shaft in the at least one image based on the position and orientation of the at least one fiducial marker;
and/or b) determining from the at least one endoscopic image that the drill bit drilled the hole in the tissue comprises determining that a marker on the drill bit is visible in a window of the shaft;
and/or c) determining from the at least one endoscopic image that the drill bit drilled the hole in the tissue comprises detecting debris created by the drill bit drilling the hole in the at least one image.

10. The system of any one of claims 7-9, wherein identifying the at least one fiducial marker disposed on the shaft of the drill guide comprises applying one or more machine learning models to the received endoscopic image to segment the drill guide in the at least one endoscopic image.

11. The system of any one of claims 7-10, wherein identifying the at least one fiducial marker comprises:
identifying one or more visual patterns of the at least one fiducial marker; and
matching the one or more visual patterns of the at least one fiducial marker to at least one fiducial marker pattern in a predefined list of fiducial marker patterns.

12. The system of any one of claims 7-11, wherein the at least one fiducial marker is disposed on a face of a fiducial ring, wherein optionally a) determining the position of the hole comprises:
selecting a corner of the face corresponding to the at least one fiducial marker;
identifying a position of the selected corner with respect to the drill guide based on the determined position and orientation of the at least one fiducial marker; and
determining a three-dimensional distance from the selected corner to a center of a distal end of the drill guide based on the identified position of the selected corner with respect to the drill guide; and/or b) determining the three-dimensional distance from the selected corner to the hole comprises:
identifying a matching corner corresponding to the selected corner, the matching corner stored in a look-up table comprising a list of fiducial markers and corresponding corners, wherein each entry pertaining to a corner in the look-up table comprises a predetermined three-dimensional distance between the corner and a position of the center of the distal end of the drill guide; and
extracting the predetermined three-dimensional distance corresponding to the identified matching corner corresponding to the selected corner in the look-up table, wherein the position of the center of the distal end of the drill guide is determined based on the predetermined three-dimensional distance.

13. A method for determining a position of a hole drilled in tissue of internal anatomy of a patient by a drill bit disposed within a drill guide, the method comprising, at a computing system:
receiving at least one endoscopic image that captures at least a distal portion of a shaft of the drill guide;
identifying in the at least one endoscopic image at least one fiducial marker disposed on the distal portion of the shaft of the drill guide;
determining a position and orientation of the at least one fiducial marker;
determining that the drill bit drilled the hole in the tissue; and
determining the position of the hole based on the position and orientation of the at least one fiducial marker.

14. The method of claim 13, further comprising displaying a visualization of the tissue that comprises a marker indicating the position of the hole, wherein optionally displaying the visualization comprises overlaying the marker on an endoscopic video and updating the position of the marker based on changes in the field of view of the endoscopic video, further optionally comprising modifying the display of the visualization of the tissue based on at least one user input.

15. The method of claim 13 or 14, wherein the at least one fiducial marker is disposed on a face of a fiducial ring, wherein optionally a) determining the position of the hole comprises:
selecting a corner of the face corresponding to the at least one fiducial marker;
identifying a position of the selected corner with respect to the drill guide based on the determined position and orientation of the at least one fiducial marker; and
determining a three-dimensional distance from the selected corner to a center of a distal end of the drill guide based on the identified position of the selected corner with respect to the drill guide; and/or b) determining the three-dimensional distance from the selected corner to the hole comprises:
identifying a matching corner corresponding to the selected corner, the matching corner stored in a look-up table comprising a list of fiducial markers and corresponding corners, wherein each entry pertaining to a corner in the look-up table comprises a predetermined three-dimensional distance between the corner and a position of the center of the distal end of the drill guide; and
extracting the predetermined three-dimensional distance corresponding to the identified matching corner corresponding to the selected corner in the look-up table, wherein the position of the center of the distal end of the drill guide is determined based on the predetermined three-dimensional distance.
